# EUROPEAN PATENT APPLICATION

(11) **EP 4 659 695 A1**
(43) Date of publication of application: **10.12.2025**
(21) Application number: 24763639.2
(22) Date of filing: 15.02.2024
(51) Int. Cl.: A61B 18/26, A61M 25/10

(54) **BALLOON CATHETER**

(30) Priority: 27.02.2023 JP 2023028925
(71) Applicant: Terumo Kabushiki Kaisha, Tokyo 151-0072 (JP)
(72) Inventor: IGUCHI, Akira, Ashigarakami-gun, Kanagawa 259-0151 (JP); KUROSAKI, Yasuo, Ashigarakami-gun, Kanagawa 259-0151 (JP)
(74) Representative: Casalonga
(86) International application number: PCT/JP2024/005324
(87) International publication number: WO 2024/181156

(57) **Abstract**

A balloon catheter according to the present disclosure includes: an elongated member; and an inflatable member supported on an outer surface of the elongated member and inflatable outward in a radial direction of the elongated member, in which from an inner side to an outer side in the radial direction, a laser emission unit that can emit a laser beam outward in the radial direction, a transmission portion that can transmit the laser beam emitted from the laser emission unit in the radial direction, and a light absorption portion that can absorb the laser beam having passed through the transmission portion are provided, and the laser emission unit, the transmission portion, and the light absorption portion are provided only in the elongated member, only in the inflatable member, or in both the elongated member and the inflatable member in a divided manner.

## Description

### Technical Field

The present disclosure relates to a balloon catheter.

### Background Art

Conventionally, there has been known a probe that converts laser beam into a shock wave and performs various treatments using the shock wave stress. Patent Literature 1 discloses this type of probe. There is also known a probe that performs treatment by converting a vaporizing inflation force of a liquid obtained by spark discharge performed in a liquid atmosphere into a mechanical force. Patent Literature 2 discloses this type of probe.

### Citation List

### Patent Literature

Patent Literature 1: JP H5-300911 A
Patent Literature 2: JP 2015-522344 A

### Summary of Invention

### Technical Problem

However, the probes described in Patent Documents 1 and 2 still have room for improvement from the viewpoint of efficiency for reliably applying a force necessary for treatment to a target site when the target site is treated, for example, crushing a calcified area in a blood vessel.

A balloon catheter is disclosed, which is capable of improving efficiency in treatment of a target site.

### Solution to Problem

A balloon catheter according to a first aspect of the present disclosure is
(1) a balloon catheter including:
   an elongated member; and
   an inflatable member supported on an outer surface of the elongated member and inflatable outward in a radial direction of the elongated member, in which
   from an inner side to an outer side in the radial direction,
   a laser emission unit that can emit a laser beam outward in the radial direction,
   a transmission portion that can transmit the laser beam emitted from the laser emission unit in the radial direction, and
   a light absorption portion that can absorb the laser beam having passed through the transmission portion are provided, and
   the laser emission unit, the transmission portion, and the light absorption portion are provided only in the elongated member, only in the inflatable member, or in both the elongated member and the inflatable member in a divided manner.
      A balloon catheter according to an embodiment of the present disclosure is
(2) the balloon catheter according to (1), in which
   the elongated member includes the laser emission unit, and
   the inflatable member includes the transmission portion and the light absorption portion.
      A balloon catheter according to an embodiment of the present disclosure is
(3) the balloon catheter according to (2), in which
   the elongated member includes
   a body member supporting the inflatable member on an outer surface, and
   a sleeve member attached to the body member so as to be rotatable in a circumferential direction relative to the body member, and
   the sleeve member includes the laser emission unit.
   A balloon catheter according to an embodiment of the present disclosure is
(4) the balloon catheter according to (3), in which the sleeve member is attached to the body member so as to be movable relative to the body member in a longitudinal direction of the elongated member.
   A balloon catheter according to an embodiment of the present disclosure is
(5) the balloon catheter according to (2), in which
   the elongated member includes
   a body member supporting the inflatable member on an outer surface, and
   a sleeve member attached to the body member,
   the sleeve member includes the laser emission unit, and
   the laser emission unit is of a full circumferential irradiation type that emits a radial laser beam.
   A balloon catheter according to an embodiment of the present disclosure is
(6) the balloon catheter according to (5), in which a portion of the sleeve member and/or the body member interposed between the laser emission unit and the inflatable member is configured to transmit a laser beam emitted from the laser emission unit.
   A balloon catheter according to an embodiment of the present disclosure is
(7) the balloon catheter according to (1), in which a conveying member that is continuous with the laser emission unit and conveys light is provided, and the conveying member has a structure that can convey laser beams of different powers.
   A balloon catheter according to an embodiment of the present disclosure is
(8) the balloon catheter according to (7), in which the conveying member is double-cladded fiber, wherein the double-cladded fiber can propagate a treatment laser beam as well as a diagnostic laser beam.
   A balloon catheter according to an embodiment of the present disclosure is
(9) the balloon catheter according to any one of (2) to (4), in which
   the inflatable member includes:
   a transmission layer as the transmission portion, the transmission layer being capable of transmitting the laser beam in the radial direction; and
   a light absorption layer as the light absorption portion that is located outward in the radial direction with respect to the transmission layer and absorbs the laser beam having passed through the transmission layer.
   A balloon catheter according to an embodiment of the present disclosure is
(10) the balloon catheter according to (1), in which the elongated member includes the laser emission unit, the transmission portion, and the light absorption portion.
   A balloon catheter according to an embodiment of the present disclosure is
(11) the balloon catheter according to (10), in which
   the elongated member includes
   a body member supporting the inflatable member on an outer surface, and
   a sleeve member attached to the body member so as to be rotatable in a circumferential direction relative to the body member, and
   the sleeve member includes the laser emission unit, the transmission portion, and the light absorption portion.
   A balloon catheter according to an embodiment of the present disclosure is
(12) the balloon catheter according to (11), in which the sleeve member is attached to the body member so as to be movable relative to the body member in a longitudinal direction of the elongated member.
   A balloon catheter according to an embodiment of the present disclosure is
(13) the balloon catheter according to (1), in which the inflatable member includes the laser emission unit, the transmission portion, and the light absorption portion.
   A balloon catheter according to an embodiment of the present disclosure is
(14) the balloon catheter according to (13), in which
   the inflatable member includes
   an inflatable body portion formed by laminating a plurality of layers, and
   a laser emission body attached to the inflatable body portion,
   the laser emission body includes the laser emission unit, and
   the inflatable body portion includes
   a transmission layer as the transmission portion, the transmission layer being capable of transmitting the laser beam in the radial direction, and
   a light absorption layer as the light absorption portion that is located outward in the radial direction with respect to the transmission layer and absorbs the laser beam having passed through the transmission layer.
   A balloon catheter according to an embodiment of the present disclosure is
(15) the balloon catheter according to any one of (1) to (14), in which the transmission portion and the light absorption portion extend over the entire area of the elongated member in a circumferential direction.
   A balloon catheter according to an embodiment of the present disclosure is
(16) the balloon catheter according to any one of (1) to (15), in which a plurality of the laser emission units is arranged at intervals in a circumferential direction of the elongated member.

### Advantageous Effects of Invention

According to the present disclosure, it is possible to provide a balloon catheter capable of improving efficiency in treating a target site.

### Brief Description of Drawings

Fig. 1 is a view illustrating a balloon catheter according to a first embodiment of the present embodiment.
Fig. 2 is a cross-sectional view of the balloon catheter illustrated in Fig. 1 taken along a plane parallel to a central axis line.
Fig. 3 is a cross-sectional view of the balloon catheter at a position of line I-I in Fig. 1.
Fig. 4 is a view illustrating a state in which an inflatable member of the balloon catheter illustrated in Fig. 1 is inflated.
Fig. 5 is a cross-sectional view of the balloon catheter illustrated in Fig. 4 at the same position as Fig. 2.
Fig. 6 is a cross-sectional view of the balloon catheter illustrated in Fig. 4 at the same position as Fig. 3.
Fig. 7 is an explanatory diagram for explaining a generation principle of laser-induced shock wave.
Fig. 8A is a cross-sectional view of a balloon catheter as a second embodiment of the present disclosure taken along a plane parallel to the central axis line.
Fig. 8B is a cross-sectional view of the balloon catheter illustrated in Fig. 8A taken along a plane orthogonal to the central axis line.
Fig. 9 is a cross-sectional view of a balloon catheter as a third embodiment of the present disclosure taken along a plane orthogonal to the central axis line.
Fig. 10 is a cross-sectional view of a balloon catheter as a fourth embodiment of the present disclosure taken along a plane parallel to the central axis line.
Fig. 11A is a cross-sectional view of the balloon catheter illustrated in Fig. 10 taken along a plane orthogonal to the central axis line.
Fig. 11B is a cross-sectional view of the balloon catheter illustrated in Fig. 10 on a plane orthogonal to the central axis line, and is a view illustrating a state in which the sleeve member has been rotated relative to the body member from the state of Fig. 11A.
Fig. 12 is a cross-sectional view of a balloon catheter as a fifth embodiment of the present disclosure taken along a plane orthogonal to the central axis line.
Fig. 13 is a view illustrating a modification of the balloon catheter as the fourth embodiment illustrated in Fig. 10.
Fig. 14 is a view illustrating another modification of the balloon catheter as the fourth embodiment illustrated in Fig. 10.
Fig. 15 is a view illustrating another modification of the balloon catheter as the fourth embodiment illustrated in Fig. 10.
Fig. 16 is a cross-sectional view of the conveying member illustrated in Fig. 15.
Fig. 17 is a view illustrating an example of a procedure performed using the balloon catheter illustrated in Fig. 15, and is a view illustrating a state in which treatment of a target site by a treatment laser beam has been completed from the state illustrated in Fig. 15.
Fig. 18 is a view illustrating an example of a procedure performed using the balloon catheter illustrated in Fig. 15, and is a view illustrating a state in which the sleeve member has been returned from the position illustrated in Fig. 17 to the position illustrated in Fig. 15.
Fig. 19 is a view illustrating an example of a procedure performed using the balloon catheter illustrated in Fig. 15, and is a view illustrating a state in which transmission and reception of a diagnostic laser beam has been completed in order to acquire a diagnosis image for a target site after treatment from the state illustrated in Fig. 18.

### Description of Embodiments

Hereinafter, an embodiment of a balloon catheter according to the present disclosure will be described as an example with reference to the drawings. In the drawings, the same components are denoted by the same reference signs.

### [First Embodiment]

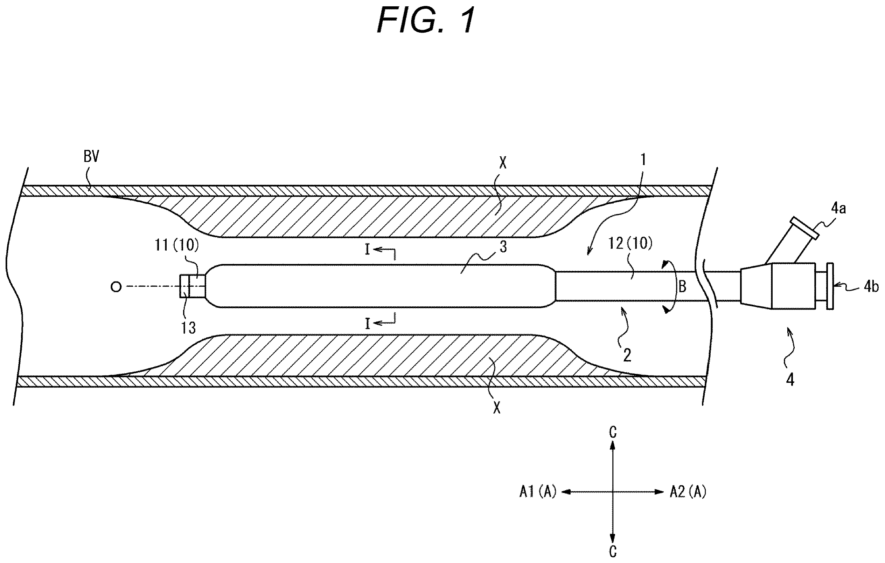

Fig. 1 is a view illustrating a balloon catheter 1 as an embodiment of the balloon catheter according to the present disclosure. Fig. 1 illustrates a state in which the balloon catheter 1 is inserted into a blood vessel BV. The balloon catheter 1 is a medical instrument that is inserted into the blood vessel BV and is capable of crushing a calcified area X in the blood vessel BV by utilizing shock waves generated by laser irradiation. In the present embodiment, as a target site to be treated by the balloon catheter 1, the calcified area X in the blood vessel BV will be exemplified and described, but the balloon catheter 1 may be used for treatment of other target sites.

As illustrated in Fig. 1, a balloon catheter 1 of the present embodiment includes an elongated member 2, an inflatable member 3, and a hub 4. Fig. 1 illustrates a state in which the balloon catheter 1 is percutaneously inserted into the blood vessel BV of a patient, and the inflatable member 3 is introduced to the position of a lesion as a target site where the calcified area X is formed. Fig. 1 shows the inflatable member 3 in a contracted state. The inflatable member 3 is guided to the lesion in the blood vessel BV in the contracted state.

Hereinafter, in the balloon catheter 1, the longitudinal direction of the elongated member 2 parallel to the central axis line O of the elongated member 2 is referred to as a "longitudinal direction A". In the balloon catheter 1, the circumferential direction of the elongated member 2 around the central axis line O of the elongated member 2 is referred to as a "circumferential direction B". Furthermore, in the balloon catheter 1, the radial direction of the elongated member 2, which is the radial direction of an imaginary circle centered on the central axis line O in an arbitrary cross section orthogonal to the central axis line O of the elongated member 2, will be referred to as "radial direction C".

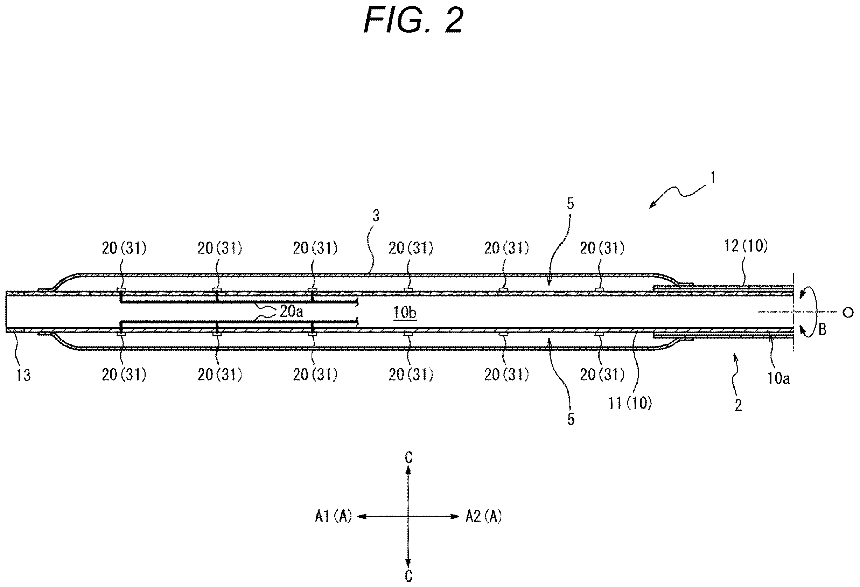

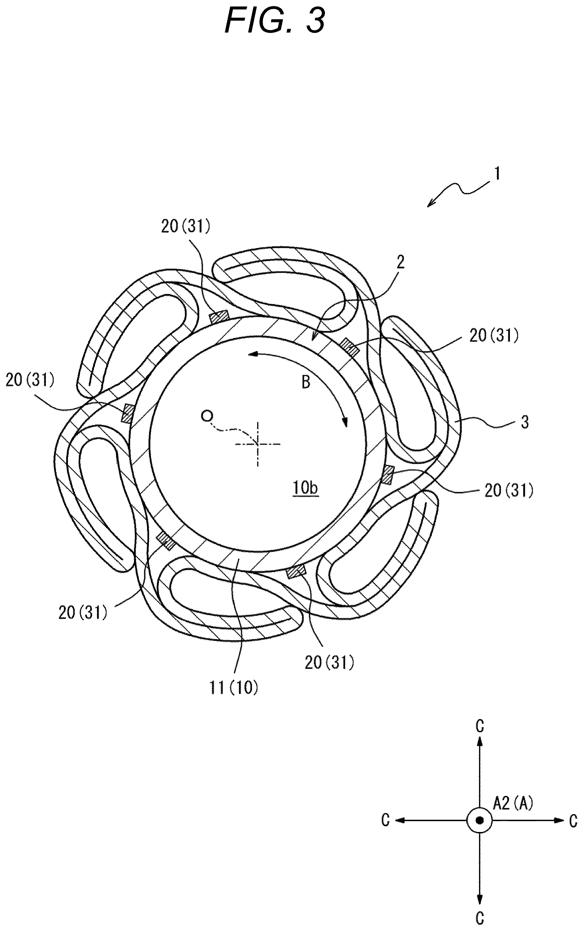

Figs. 2 and 3 are cross-sectional views of the balloon catheter 1 illustrated in Fig. 1. Fig. 2 is a cross-sectional view of the balloon catheter 1 in a plane including the central axis line O and parallel to the central axis line O. Fig. 2 illustrates only an end on the distal side (hereinafter referred to as a "distal end portion") of the balloon catheter 1. Fig. 3 is a cross-sectional view of the balloon catheter 1 taken along line I-I in Fig. 1.

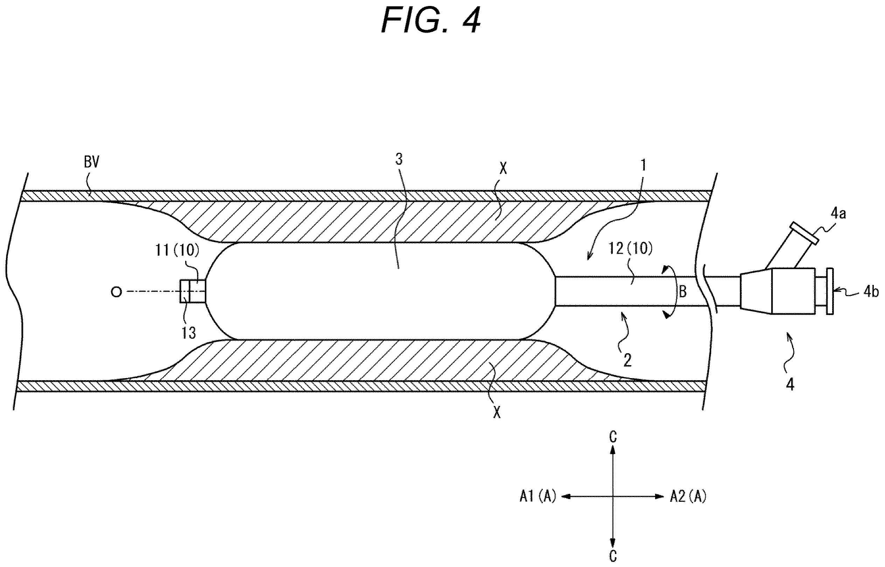

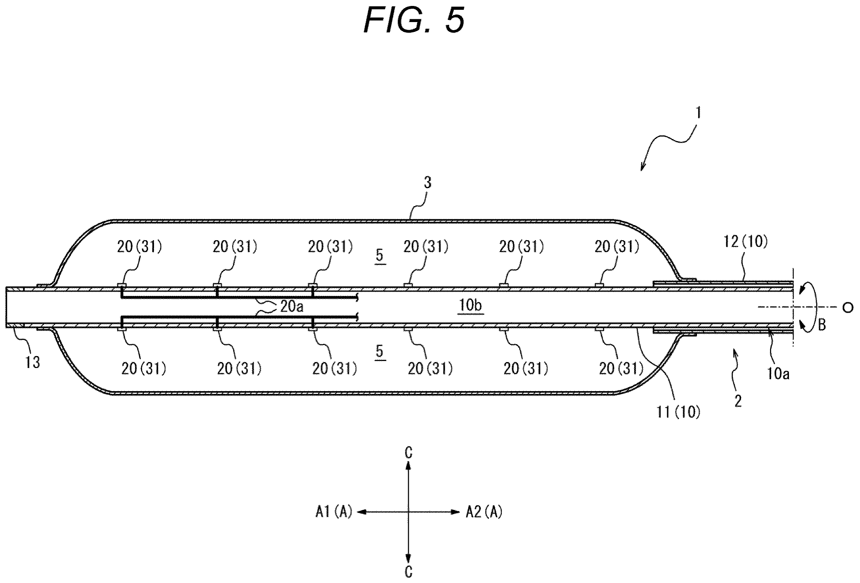

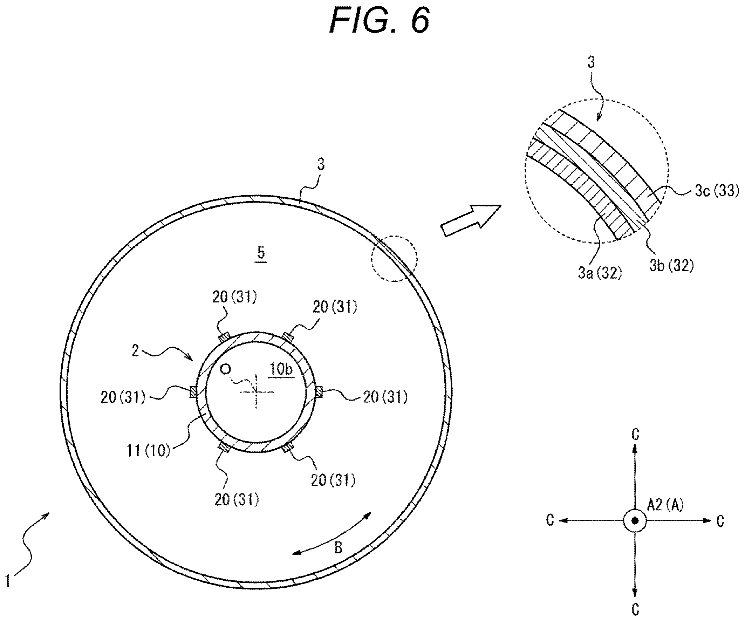

Figs. 4 to 6 show a state in which the inflatable member 3 in the contracted state shown in Figs. 1 to 3 is inflated. Specifically, Fig. 4 illustrates a state in which the inflatable member 3 in the contracted state illustrated in Fig. 1 is inflated in the blood vessel BV. Fig. 5 is a cross-sectional view at the same position as Fig. 2, and illustrates the inflatable member 3 in the inflated state. Fig. 6 is a cross-sectional view at the same position as Fig. 3, and illustrates the inflatable member 3 in the inflated state.

As shown in Figs. 1 to 6, the inflatable member 3 is supported on the outer surface of the elongated member 2 and is inflatable outward in the radial direction C of the elongated member 2.

As described later in detail, the balloon catheter 1 is provided with a laser emission unit 31, a transmission portion 32, and a light absorption portion 33 from the inside to the outside in the radial direction C. The laser emission unit 31 is configured to emit a laser beam outward in the radial direction C. The transmission portion 32 is configured to transmit the laser beam emitted from the laser emission unit 31 in the radial direction C. The light absorption portion 33 can absorb the laser beam transmitted through the transmission portion 32. The laser beam emitted from the laser emission unit 31 passes through the transmission portion 32 and is absorbed by the light absorption portion 33. In the light absorption portion 33, plasma is generated by the absorbed laser beam. The plasma generated in the light absorption portion 33 is likely to stay inside the light absorption portion 33 due to the transmission portion 32 covering the inside of the light absorption portion 33 in the radial direction C. As a result, the laser-induced shock wave can be transmitted from the light absorption portion 33 toward the outside in the radial direction C. In the balloon catheter 1, it is possible to crush the calcified area X by applying the laser-induced shock wave to the calcified area X in the blood vessel BV.

In addition, in the balloon catheter 1, a state in which the inflatable member 3 is in contact with the calcified area X which is a target site can be realized. Therefore, the above-described laser-induced shock wave can reliably act on the calcified area X in the blood vessel BV. That is, with the balloon catheter 1, by using the inflatable member 3 while ensuring the force necessary for the treatment of the target site by using the laser-induced shock wave, the efficiency in performing the treatment of the target site can be improved by reliably applying the laser-induced shock wave to the target site.

The laser emission unit 31 only needs to be able to emit a laser beam capable of generating a laser-induced shock wave in the light absorption portion 33, and for example, a nanosecond pulse laser, a picosecond laser, or a femtosecond pulse laser can be used.

The transmission portion 32 is not particularly limited as long as it can transmit the laser beam emitted from the laser emission unit 31. The transmission portion 32 can be exemplified by, for example, a transparent portion formed of a polymer material such as polyolefins (for example, polyethylene, polypropylene, polybutene, ethylene-propylene copolymer, ethylene-vinyl acetate copolymer, ionomer, or a mixture of two or more types of the polyolefins listed above), polyvinyl chloride, polyamide, polyamide elastomer, polyurethane, polyurethane elastomer, polyimide, or fluororesin, or a mixture of the polymer materials listed above. The thickness of the transmission portion 32 can be, for example, 1 µm to -500 µm. However, the thickness of the transmission portion 32 is preferably 5 µm to -100 µm, and more preferably 10 µm to -50 µm.

The configuration of the light absorption portion 33 is not particularly limited as long as it can absorb the laser beam emitted from the laser emission unit 31 and transmitted through the transmission portion 32. The light absorption portion 33 may be made of, for example, black rubber such as EPDM (Ethylene Propylene Diene Monomer) which is natural rubber or synthetic rubber, nitrile, chloroprene, or neoprene, or flexible resin in which a black component such as carbon black or black perylene pigment is blended. The thickness of the light absorption portion 33 can be, for example, 1 µm to -500 µm. However, the thickness of the light absorption portion 33 is preferably 5 µm to -100 µm, and more preferably 10 µm to - 50 µm.

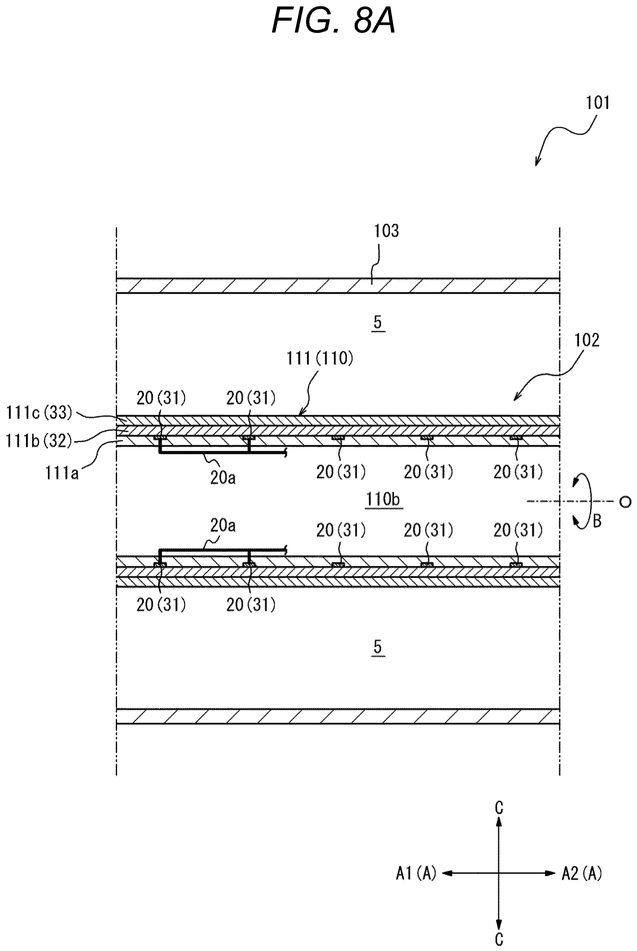

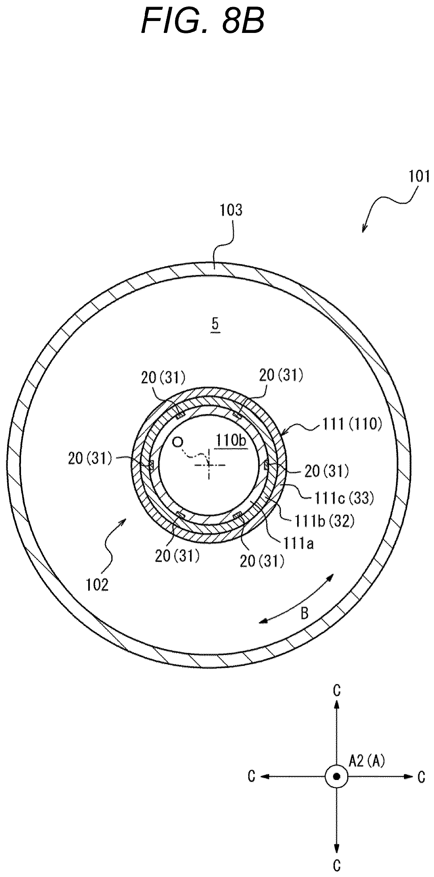

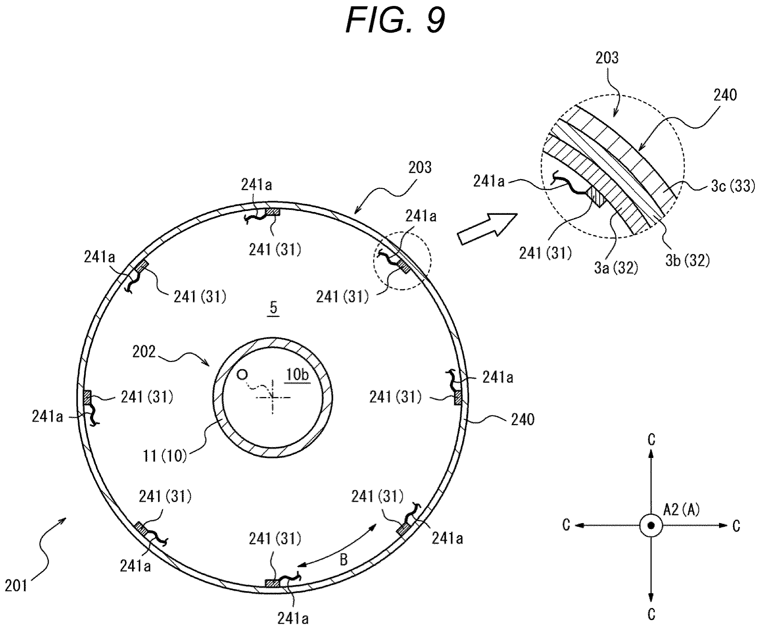

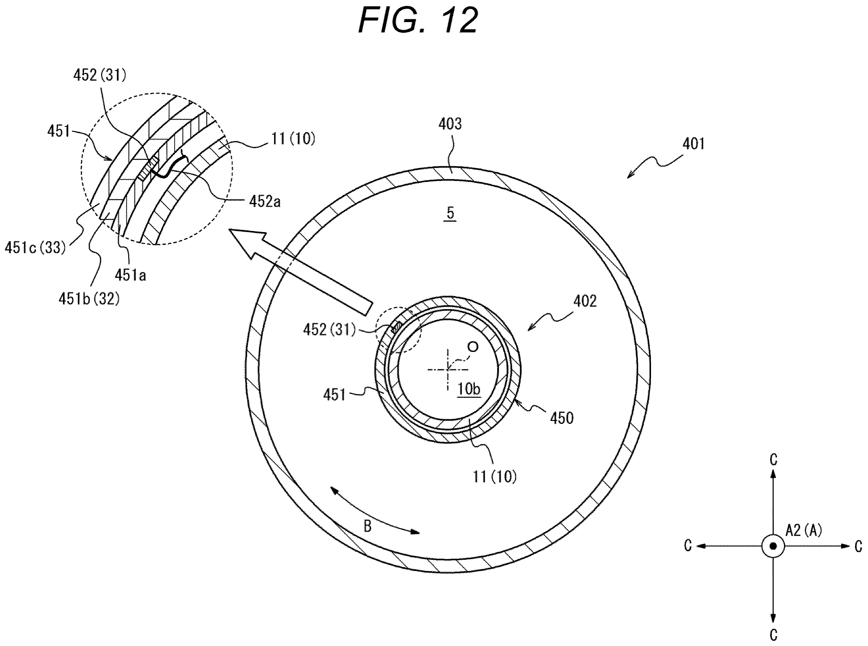

The laser emission unit 31, the transmission portion 32, and the light absorption portion 33 described above are provided only in the elongated member 2, only in the inflatable member 3, or separately in both the elongated member 2 and the inflatable member 3. In the balloon catheter 1 of the present embodiment, the laser emission unit 31, the transmission portion 32, and the light absorption portion 33 are provided separately for both the elongated member 2 and the inflatable member 3. A configuration in which the laser emission unit 31, the transmission portion 32, and the light absorption portion 33 are provided only in the elongated member will be described later (see Figs. 8A, 8B, and 12). Further, a configuration in which the laser emission unit 31, the transmission portion 32, and the light absorption portion 33 are provided only in the inflatable member will be described later (see Fig. 9).

Hereinafter, details of the balloon catheter 1 of the present embodiment will be described.

### <Elongated member 2>

As illustrated in Figs. 1 and 4, the elongated member 2 is percutaneously inserted into the blood vessel BV of the patient from the distal end thereof. A hub 4 is connected to a proximal end of the elongated member 2. Hereinafter, in the longitudinal direction A, the direction from the proximal end side toward the distal end side of the elongated member 2 is simply referred to as "distal direction A1" or "distal side". In the longitudinal direction A, a direction opposite to the distal direction A1, which is a direction from the distal end side to the proximal end side of the elongated member 2, is simply referred to as a "proximal direction A2" or a "proximal side".

The elongated member 2 of the present embodiment includes an elongated body member 10 and a laser emission body 20 attached to the body member 10. As illustrated in Figs. 2, 3, 5, and 6, the elongated member 2 of the present embodiment includes a plurality of laser emission bodies 20 arranged at different positions in the longitudinal direction A and the circumferential direction B of the body member 10.

The body member 10 supports the inflatable member 3. More specifically, the body member 10 supports the inflatable member 3 at the distal end portion of the body member 10. The body member 10 internally defines a flow path 10a capable of supplying a fluid to the accommodation space 5 defined by the inflatable member 3. The fluid supplied to the accommodation space 5 of the inflatable member 3 can be taken out through the flow path 10a by suction or the like. The flow path 10a extends from an end portion (hereinafter, the proximal end portion will be referred to as a "proximal end portion") on the proximal end side connected to the hub 4 of the body member 10 to a position where the inflatable member 3 in the longitudinal direction A is provided. A proximal end of the flow path 10a communicates with an in-hub flow path of the hub 4. As illustrated in Figs. 2 and 5, the distal end of the flow path 10a communicates with the accommodation space 5.

As illustrated in Figs. 2, 3, 5, and 6, the body member 10 internally defines a guide wire insertion hole 10b through which a guide wire can be inserted. The body member 10 is guided in the blood vessel BV along the guide wire inserted into the guide wire insertion hole 10b. The guide wire insertion hole 10b extends from the proximal end of the body member 10 to a distal opening defined by the distal end surface of the body member 10.

As illustrated in Figs. 2 and 5, the body member 10 of the present embodiment includes an inner tube 11 that defines the guide wire insertion hole 10b, and an outer tube 12 that covers the outer side of the inner tube 11 in the radial direction C and is disposed concentrically with the inner tube 11. The inner tube 11 is disposed to protrude further in the distal direction A1 than the distal end of the outer tube 12. A marker member 13 is attached to the distal end portion of the inner tube 11. The marker member 13 has X-ray detectability. Specifically, the marker member 13 is made of a material having high radiopacity. Specifically, the marker member 13 can be made of a material having high radiopacity such as platinum, gold, iridium, or tungsten. The flow path 10a of the present embodiment is defined between the outer surface of the inner tube 11 and the inner surface of the outer tube 12. The flow path 10a communicates with the accommodation space 5 at the position of the distal end of the outer tube 12. However, the configuration of the body member 10 is not limited to the configuration of the present embodiment. The body member 10 of the present embodiment is realized by forming the flow path 10a and the guide wire insertion hole 10b into a double tube structure, but a means for realizing the flow path 10a and the guide wire insertion hole 10b is not limited to the double tube structure.

As a material for forming the inner tube 11 and the outer tube 12 of the body member 10, for example, thermoplastic resins such as polyolefin (for example, polyethylene and polypropylene), polyolefin elastomer (for example, an elastomer using a polyethylene elastomer, a polypropylene elastomer, an ethylene-propylene copolymer, or the like), polyvinyl chloride, ethylene-vinyl acetate copolymer, polyamide elastomer, polyurethane, and fluororesin, silicone rubber, and the like can be used.

In the present embodiment, the elongated member 2 includes the laser emission unit 31. More specifically, in the present embodiment, the laser emission body 20 of the elongated member 2 includes the laser emission unit 31. The laser emission body 20 may be, for example, a nanosecond pulse laser or a femtosecond pulse laser. As illustrated in Figs. 2, 3, 5, and 6, the laser emission body 20 of the present embodiment is attached to the body member 10. More specifically, the laser emission body 20 of the present embodiment is attached on the outer surface of the inner tube 11 of the body member 10 at the position where the inflatable member 3 is provided in the longitudinal direction A. The laser emission unit 31 of the laser emission body 20 can emit a laser beam from the outer surface of the inner tube 11 toward the outside in the radial direction C.

As illustrated in Figs. 2, 3, 5, and 6, it is preferable that only the accommodation space 5 is interposed between the laser emission unit 31 and the inflatable member 3 in the radial direction C, and no other member or site of the balloon catheter 1 is interposed between the laser emission unit 31 and the inflatable member 3 in the radial direction. In this manner, the laser beam emitted from the laser emission unit 31 toward the outside in the radial direction C is emitted to the inflatable member 3 without being attenuated by other members or sites of the balloon catheter 1 interposed between the laser emission unit 31 and the inflatable member 3.

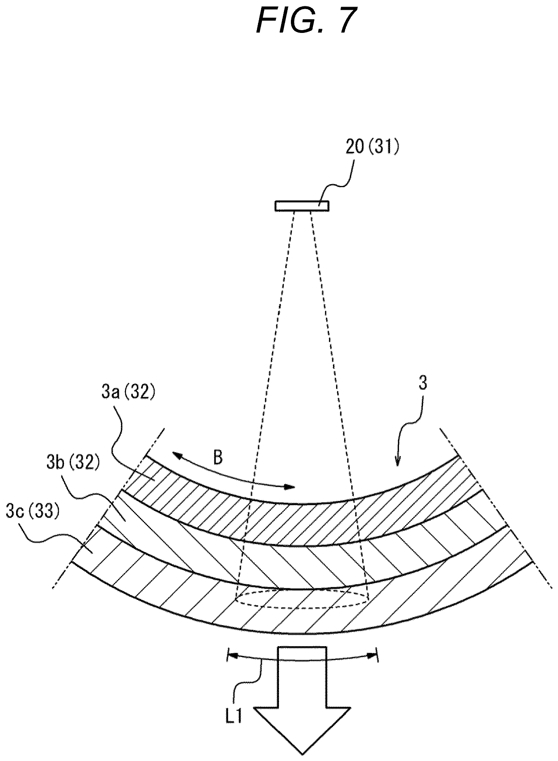

As illustrated in Figs. 3 and 6, a plurality of laser emission bodies 20 including the laser emission unit 31 is disposed at intervals in the circumferential direction B of the elongated member 2. In this way, the laser beam can be emitted from the plurality of laser emission units 31 of the plurality of laser emission bodies 20 toward the outside in the radial direction C in a wide range in the circumferential direction B. As a result, the laser-induced shock wave can more reliably act on the calcified area X not only when the calcified area X is formed over the entire circumferential area of the inner wall of the blood vessel BV but also when the calcified area X is formed only in a part of the circumferential area of the inner wall of the blood vessel BV. An irradiation range L1 (see Fig. 7) in the circumferential direction B of the light absorption portion 33 to which the laser beam from each laser emission unit 31 is applied may be appropriately set. Therefore, by appropriately setting the number of laser emission units 31 in the circumferential direction B and the irradiation range in the circumferential direction B by each laser emission unit 31 in the light absorption portion 33, the laser-induced shock wave can be sent outward in the radial direction C in a desired range in the circumferential direction B.

As illustrated in Figs. 2 and 5, a plurality of laser emission bodies 20 including the laser emission unit 31 is disposed at intervals in the longitudinal direction A of the elongated member 2. In this way, the laser beam can be emitted from the plurality of laser emission units 31 of the plurality of laser emission bodies 20 toward the outside in the radial direction C in a wide range in the longitudinal direction A. As a result, the laser-induced shock wave can more reliably act on the calcified area X not only when the calcified area X is formed in a wide range in the extending direction of the blood vessel BV but also when the calcified area X is formed only in a narrow range in the extending direction of the blood vessel BV. The laser emission body 20 may include a laser emission unit 31 that is elongated in the longitudinal direction A and is capable of emitting a wide laser having uniform intensity in the radial direction C regardless of the position in the longitudinal direction A. In such a case, a plurality of laser emission bodies 20 may not be disposed in the longitudinal direction A.

As illustrated in Figs. 2 and 5, the laser emission body 20 includes a conveying member 20a including an optical fiber that transmits light to the laser emission unit 31. The arrangement position of the conveying member 20a is not particularly limited, but the conveying member 20a may be disposed along the inner surface of the inner tube 11 of the body member 10 as illustrated in Figs. 2 and 5. Furthermore, the conveying member 20a may be disposed, for example, along the outer surface of the inner tube 11 or along an insertion hole formed in the peripheral wall of the inner tube 11. In Figs. 3 and 6, the conveying member 20a is not illustrated.

### <Inflatable member 3>

As shown in Figs. 2 and 5, the inflatable member 3 is supported on the outer surface of the elongated member 2. Specifically, the inflatable member 3 of the present embodiment is supported on the outer surface of the body member 10 of the elongated member 2. More specifically, the inflatable member 3 of the present embodiment is supported across the outer surface of the inner tube 11 of the body member 10 and the outer surface of the outer tube 12 of the body member 10 so as to straddle the distal end of the outer tube 12 in the longitudinal direction A.

The inflatable member 3 of the present embodiment is supported on the outer surface of the elongated member 2 in a state of surrounding the outer side of the elongated member 2 in the radial direction C. That is, the inflatable member 3 surrounds the outer side of the outer surface of the elongated member 2 in the radial direction C over the entire region of the elongated member 2 in the circumferential direction B.

The inflatable member 3 is inflatable outward in the radial direction C of the elongated member 2. More specifically, the inflatable member 3 of the present embodiment is formed of an extension membrane body attached to the outer surface of the body member 10 of the elongated member 2. Both end portions in the longitudinal direction A of the inflatable membrane body as the inflatable member 3 are annularly joined to the outer surface of the elongated member 2 by adhesion, fusion, or the like over the entire region in the circumferential direction B of the elongated member 2. More specifically, the distal end of the inflatable membrane body as the inflatable member 3 is annularly joined to the outer surface of the inner tube 11 over the entire region in the circumferential direction B. In addition, the proximal end of the inflatable membrane body as the inflatable member 3 is annularly joined to the outer surface of the outer tube 12 over the entire region in the circumferential direction B. The central portion in the longitudinal direction A of the inflatable membrane body as the inflatable member 3 is not bonded onto the outer surfaces of the inner tube 11 and the outer tube 12 over the entire region in the circumferential direction B of the elongated member 2, and the annular accommodation space 5 is defined between the central portion and the outer surface of the elongated member 2. When the fluid is supplied to the accommodation space 5 through the flow path 10a of the elongated member 2 described above, the inflatable membrane body as the inflatable member 3 is pressed by the fluid and inflates outward in the radial direction C of the elongated member 2 in the entire region in the circumferential direction B of the elongated member 2.

As shown in Fig. 3, the inflatable membrane body as the inflatable member 3 is wound along the outer surface of the elongated member 2 in a folded state in a contracted state. When the fluid is supplied to the accommodation space 5, the inflatable membrane body as the inflatable member 3 in the contracted state inflates so as to protrude outward in the radial direction C of the elongated member 2 so that the fold line inflates. As a result, as shown in Fig. 6, the inflatable membrane body as the inflatable member 3 is in an inflated form.

The fluid supplied to the accommodation space 5 may be a gas or a liquid, and examples of the gas or the liquid can include gases such as helium gas, CO₂ gas, and O₂ gas, and liquids such as saline (or saline solution) and contrast media.

The inflatable member 3 of the present embodiment is formed of an extension membrane body attached on the outer surface of the body member 10 of the elongated member 2, but the present disclosure is not limited to this configuration. The inflatable member 3 may be an annular bag body supported on the outer surface of the body member 10 of the elongated member 2. That is, the accommodation space 5 of the inflatable member 3 may be a space defined only by the bag body as the inflatable member 3. As described above, the inflatable member 3 only needs to be able to form a balloon that can be inflated and contracted by a fluid, and the inflatable member 3 may be formed of an inflatable membrane body or a bag body. However, when the inflatable member 3 is formed of a bag body, the film portion on the inner side in the radial direction C of the bag body forming the inflatable member 3 is interposed between the laser emission unit 31 and the film portion on the outer side in the radial direction C of the bag body forming the inflatable member 3. Therefore, it is necessary to configure the film portion on the inner side in the radial direction C of the bag body constituting the inflatable member 3 so that the laser beam can pass through the film portion on the inner side in the radial direction C. As a result, the laser beam emitted from the laser emission unit 31 reaches the film portion on the outer side in the radial direction C in the bag body constituting the inflatable member 3. Therefore, from the viewpoint of simplifying the configuration of the inflatable member 3, the inflatable member 3 is preferably formed of an extension membrane body as in the present embodiment.

As illustrated in Fig. 6, the inflatable member 3 of the present embodiment includes the transmission portion 32 and the light absorption portion 33 described above. Specifically, the inflatable member 3 of the present embodiment includes a first transmission layer 3a and a second transmission layer 3b as the transmission portion 32. In addition, the inflatable member 3 of the present embodiment includes the light absorption layer 3c as the light absorption portion 33 that is located on the outer side in the radial direction C with respect to the first transmission layer 3a and the second transmission layer 3b and absorbs the laser transmitted through the first transmission layer 3a and the second transmission layer 3b.

In the present embodiment, the first transmission layer 3a and the second transmission layer 3b are laminated in the order of the first transmission layer 3a and the second transmission layer 3b from the inside to the outside in the radial direction C. The first transmission layer 3a and the second transmission layer 3b may be, for example, transparent resin layers. The second transmission layer 3b may be, for example, a base material layer of an inflatable membrane body constituting the inflatable member 3. The first transmission layer 3a may be, for example, an inner surface layer constituting an inner surface in the radial direction C of the inflatable membrane body constituting the inflatable member 3. The inner surface layer as the first transmission layer 3a may be disposed for protecting the inner surface of the inflatable membrane body, flexibility, and the like.

Examples of the material of the first transmission layer 3a and the second transmission layer 3b include polyolefins such as polyethylene, polypropylene, and ethylene-propylene copolymers, polyesters such as polyethylene terephthalate, thermoplastic resins such as polyvinyl chloride, ethylene-vinyl acetate copolymers, crosslinked ethylene-vinyl acetate copolymers, and polyurethane, and polyamides.

The light absorption layer 3c may be, for example, a black rubber layer, a black resin layer, or the like. The light absorption layer 3c is an outer surface layer constituting an outer surface in the radial direction C of the inflatable membrane body constituting the inflatable member 3.

As described above, the inflatable member 3 of the present embodiment includes the first transmission layer 3a and the second transmission layer 3b as the transmission portion 32, and the light absorption layer 3c as the light absorption portion 33. The first transmission layer 3a, the second transmission layer 3b, and the light absorption layer 3c are disposed in the order of the first transmission layer 3a, the second transmission layer 3b, and the light absorption layer 3c from the inside to the outside in the radial direction C. As a result, as illustrated in Fig. 7, the laser beam emitted from the laser emission unit 31 of the elongated member 2 described above passes through the fluid stored in the accommodation space 5 and the first transmission layer 3a and the second transmission layer 3b of the inflatable member 3, and is absorbed by the light absorption layer 3c of the inflatable member 3. In the light absorption layer 3c, plasma is generated by the absorbed laser beam. The plasma generated in the light absorption layer 3c is likely to stay in the light absorption layer 3c by the first transmission layer 3a and the second transmission layer 3b covering the inside of the light absorption layer 3c in the radial direction C. As a result, a laser-induced shock wave can be sent from the light absorption layer 3c toward the outside in the radial direction C, that is, the outside of the inflatable member 3. In the balloon catheter 1 of the present embodiment, it is possible to crush the calcified area X by applying the laser-induced shock wave to the calcified area X in the blood vessel BV.

Therefore, it is preferable that at least one of the first transmission layer 3a and the second transmission layer 3b as the transmission portion 32 and the light absorption layer 3c as the light absorption portion 33 extend over the entire region in the circumferential direction B of the elongated member 2. In this way, in a wider range in the circumferential direction B, the laser-induced shock wave can be sent from the light absorption portion 33 to the outside in the radial direction C.

In addition, as in the present embodiment, since the inflatable member 3 includes the transmission portion 32 and the light absorption portion 33, as compared with a configuration in which the transmission portion 32 and the light absorption portion 33 are provided in the elongated member, the laser-induced shock wave sent to the outside in the radial direction C from the light absorption portion 33 can act on the calcified area X (see Fig. 1 and the like) without being attenuated. Therefore, from the viewpoint of suppressing attenuation of the laser-induced shock wave, the transmission portion 32 and the light absorption portion 33 are preferably provided in the inflatable member 3.

The inflatable member 3 of the present embodiment includes the first transmission layer 3a and the second transmission layer 3b as the transmission portion 32, but is not limited to this configuration. The inflatable member 3 may include only one transmission layer as the transmission portion 32. In addition, the inflatable member 3 of the present embodiment does not include another layer on the inner side in the radial direction C than the first transmission layer 3a and the second transmission layer 3b as the transmission portion 32, but is not limited to this configuration. Another transmission layer capable of transmitting the laser beam emitted from the laser emission unit 31 may be further provided. In other words, the inflatable member 3 includes only one or a plurality of transmission layers inside the light absorption layer 3c in the radial direction C.

Further, in the inflatable member 3 of the present embodiment, the light absorption layer 3c as the light absorption portion 33 is the outer surface layer of the inflatable member 3, but another transmission layer may be laminated on the outer side in the radial direction C. However, as in the present embodiment, the light absorption layer 3c is preferably an outer surface layer of the inflatable member 3. In this way, it is possible to suppress attenuation of the laser-induced shock wave by another transmission layer outside the light absorption layer 3c in the radial direction C.

### <Hub 4>

As illustrated in Figs. 1 and 4, the elongated member 2 is connected to the distal side of the hub 4. In the hub 4, an in-hub flow path communicating with the flow path 10a of the body member 10 of the elongated member 2 is defined. The fluid can be supplied from the connection portion 4a provided on the proximal side of the hub 4 to the flow path 10a of the body member 10 of the elongated member 2 through the in-hub flow path. In the hub 4, an in-hub insertion hole communicating with the guide wire insertion hole 10b of the body member 10 of the elongated member 2 is defined. The guide wire can be inserted into the guide wire insertion hole 10b of the body member 10 of the elongated member 2 from the proximal opening 4b, which is the proximal end of the hub insertion hole provided on the proximal side of the hub 4, through the hub insertion hole.

### [Second Embodiment]

Next, a balloon catheter 101 as another embodiment of the balloon catheter according to the present disclosure will be described with reference to Figs. 8A and 8B. Here, differences of the balloon catheter 101 from the balloon catheter 1 of the above-described first embodiment (see Fig. 1 and the like) will be described, and description of common configurations will be omitted.

In the balloon catheter 101 of the present embodiment, the laser emission unit 31, the transmission portion 32, and the light absorption portion 33 are provided only in the elongated member 102. Fig. 8A is an enlarged cross-sectional view illustrating a position of the inflatable member 103 in a cross section of the balloon catheter 101 in a plane including the central axis line O and parallel to the central axis line O in an enlarged manner. Fig. 8B is a cross-sectional view of the balloon catheter 101 in a plane orthogonal to the central axis line O at the position of the inflatable member 103. Figs. 8A and 8B both illustrate the inflatable member 103 in an inflated state.

As illustrated in Figs. 8A and 8B, the elongated member 102 of the present embodiment includes a laser emission unit 31, a transmission portion 32, and a light absorption portion 33. More specifically, the elongated member 102 of the present embodiment includes a body member 110 and a laser emission body 20. The body member 110 of the elongated member 102 includes a laser emission unit 31, a transmission portion 32, and a light absorption portion 33.

The body member 110 includes an inner tube 111 and an outer tube 12. Although the outer tube 12 is not illustrated in Figs. 8A and 8B, the configuration of the outer tube 12 is similar to that of the first embodiment described above (see Fig. 2 and the like). The inner tube 111 is different from the inner tube 11 (see Fig. 3 and the like) of the first embodiment described above in that it includes a transmission layer 111b as the transmission portion 32 and a light absorption layer 111c as the light absorption portion 33, and other configurations are the same. The inner tube 111 defines a guide wire insertion hole 110b in the inner tube 111.

Specifically, the inner tube 111 of the present embodiment includes a base material layer 111a, a transmission layer 111b laminated on the outer side of the base material layer 111a in the radial direction C, and a light absorption layer 111c laminated on the outer side of the transmission layer 111b in the radial direction C.

The base material layer 111a may be made of, for example, the materials exemplified as the materials for forming the inner tube 11 (see Fig. 3 and the like) of the first embodiment described above.

The transmission layer 111b may be made of, for example, the materials exemplified as the materials for forming the first transmission layer 3a and the second transmission layer 3b (see Fig. 6) of the inflatable member 3 of the first embodiment described above.

The light absorption layer 111c may be made of, for example, the materials exemplified as the materials for forming the light absorption layer 3c (see Fig. 6) of the inflatable member 3 of the first embodiment described above.

As illustrated in Figs. 8A and 8B, the laser emission body 20 is embedded in the peripheral wall of the inner tube 11. More specifically, the laser emission body 20 of the present embodiment is sandwiched between the base material layer 111a and the transmission layer 111b. The laser emission unit 31 of the laser emission body 20 can emit a laser beam from the outer surface of the base material layer 111a of the inner tube 111 toward the outside in the radial direction C.

As illustrated in Fig. 8B, a plurality of laser emission bodies 20 including the laser emission unit 31 is disposed at intervals in the circumferential direction B of the elongated member 102. In this way, the laser beam can be emitted from the plurality of laser emission units 31 of the plurality of laser emission bodies 20 toward the outside in the radial direction C in a wide range in the circumferential direction B. As a result, the laser-induced shock wave can more reliably act on the calcified area X not only when the calcified area X is formed over the entire circumferential area of the inner wall of the blood vessel BV but also when the calcified area X is formed only in a part of the circumferential area of the inner wall of the blood vessel BV.

As illustrated in Fig. 8A, a plurality of laser emission bodies 20 including the laser emission unit 31 is disposed at intervals in the longitudinal direction A of the elongated member 102. In this way, the laser beam can be emitted from the plurality of laser emission units 31 of the plurality of laser emission bodies 20 toward the outside in the radial direction C in a wide range in the longitudinal direction A. As a result, the laser-induced shock wave can more reliably act on the calcified area X not only when the calcified area X is formed in a wide range in the extending direction of the blood vessel BV but also when the calcified area X is formed only in a narrow range in the extending direction of the blood vessel BV. The laser emission body 20 may include a laser emission unit 31 that is long in the longitudinal direction A and is capable of emitting a wide laser having uniform intensity in the radial direction C regardless of the position in the longitudinal direction A. In such a case, a plurality of laser emission bodies 20 may not be disposed in the longitudinal direction A.

As illustrated in Fig. 8A, the laser emission body 20 includes a conveying member 20a including an optical fiber that transmits light to the laser emission unit 31. The arrangement position of the conveying member 20a is not particularly limited, but the conveying member 20a may be disposed along the inner surface of the inner tube 111 as illustrated in Fig. 8A. Further, for example, the conveying member 20a may be disposed along an insertion hole formed in the peripheral wall of the inner tube 111. In Fig. 8B, the conveying member 20a is not illustrated.

The laser beam emitted from the laser emission unit 31 of the laser emission body 20 passes through the transmission layer 111b and is absorbed by the light absorption layer 111c. In the light absorption layer 111c, plasma is generated by the absorbed laser beam. The plasma generated in the light absorption layer 111c is likely to stay in the light absorption layer 111c by the transmission layer 111b covering the inside of the light absorption layer 111c in the radial direction C. As a result, a laser-induced shock wave can be sent from the light absorption layer 111c toward the outside in the radial direction C, that is, the outside of the elongated member 102. The laser-induced shock wave propagates through the fluid accommodated in the accommodation space 5 and the inflatable member 103, and is sent to the outside of the inflatable member 103 in the radial direction C. In this manner, in the balloon catheter 101 of the present embodiment, it is possible to crush the calcified area X by applying the laser-induced shock wave to the calcified area X (see Fig. 1 and the like) in the blood vessel BV (see Fig. 1 and the like).

In the present embodiment, the transmission portion 32 and the light absorption portion 33 are provided in the elongated member 102 on the inner side in the radial direction C with respect to the inflatable member 103. Therefore, the peripheries of the transmission portion 32 and the light absorption portion 33 are protected by the inflatable member 103. Therefore, it is possible to prevent the transmission portion 32 and the light absorption portion 33 from being damaged by inflation and contraction of the inflatable member 103, contact between the inflatable member 103 and the inner wall of the blood vessel BV (see Fig. 1 and the like) or the calcified area X (see Fig. 1 and the like. As described above, from the viewpoint of the protection of the transmission portion 32 and the light absorption portion 33, the transmission portion 32 and the light absorption portion 33 are preferably provided in the elongated member 102.

The transmission layer 111b as the transmission portion 32 and the light absorption layer 111c as the light absorption portion 33 preferably extend over the entire region in the circumferential direction B of the elongated member 102. In this way, the laser-induced shock wave can be sent from the light absorption portion 33 to the outside in the radial direction C regardless of the arrangement position of the laser emission unit 31 in the circumferential direction B.

The inner tube 111 of the present embodiment has a three-layer configuration including the base material layer 111a, the transmission layer 111b, and the light absorption layer 111c, but is not limited to this configuration. The inner tube 111 may have, for example, a two-layer configuration including only the transmission layer 111b and the light absorption layer 111c. In such a case, the laser emission body 20 may be attached to the inner surface of the transmission layer 111b constituting the inner surface layer of the inner tube 111. Furthermore, the inner tube 111 may have, for example, a configuration of four or more layers including the transmission layer 111b and the light absorption layer 111c. Even in such a case, the inner tube 111 includes only at least one transmission layer including the transmission layer 111b inside the light absorption layer 111c in the radial direction C, and does not include layers other than the transmission layer. Further, in the inner tube 111 of the present embodiment, the light absorption layer 111c as the light absorption portion 33 is the outer surface layer of the inner tube 111, but another transmission layer may be laminated on the outer side in the radial direction C. However, as in the present embodiment, the light absorption layer 111c is preferably an outer surface layer of the inner tube 111. In this way, it is possible to suppress attenuation of the laser-induced shock wave by another transmission layer outside the light absorption layer 111c in the radial direction C.

In addition, the inflatable member 103 of the present embodiment may have a configuration capable of propagating the laser-induced shock wave transmitted from the light absorption layer 111c of the elongated member 102 from the inside to the outside in the radial direction C, and the configuration is not particularly limited. Examples of the material of the inflatable member 103 include polyolefins such as polyethylene, polypropylene, and ethylene-propylene copolymer, polyesters such as polyethylene terephthalate, thermoplastic resins such as polyvinyl chloride, ethylene-vinyl acetate copolymer, crosslinked ethylene-vinyl acetate copolymer, and polyurethane, polyamide, polyamide elastomer, silicone rubber, latex rubber, and the like.

### [Third Embodiment]

Next, a balloon catheter 201 as another embodiment of the balloon catheter according to the present disclosure will be described with reference to Fig. 9. Here, differences of the balloon catheter 201 from the balloon catheter 1 of the above-described first embodiment (see Fig. 1 and the like) will be described, and description of common configurations will be omitted.

In the balloon catheter 201 of the present embodiment, the laser emission unit 31, the transmission portion 32, and the light absorption portion 33 are provided only in the inflatable member 203. Fig. 9 is a cross-sectional view of the balloon catheter 201 in a plane orthogonal to the central axis line O at the position of the inflatable member 203. Fig. 9 illustrates the inflatable member 203 in an inflated state.

As illustrated in Fig. 9, the elongated member 202 of the present embodiment is configured by a body member 10. In other words, the elongated member 202 of the present embodiment is different from the elongated member 2 (see Fig. 2 and the like) of the first embodiment described above only in the presence or absence of the laser emission body 20. That is, the elongated member 202 of the present embodiment does not include the laser emission body 20.

On the other hand, the inflatable member 203 of the present embodiment includes a laser emission unit 31, a transmission portion 32, and a light absorption portion 33. More specifically, the inflatable member 203 of the present embodiment includes an inflatable body portion 240 and a laser emission body 241 attached to the inflatable body portion 240.

The inflatable body portion 240 has the same configuration as the inflatable member 3 (see Fig. 6) of the first embodiment described above. That is, the inflatable body portion 240 is supported on the outer surface of the elongated member 202, and is inflatable outward in the radial direction C by supplying a fluid to the accommodation space 5. As illustrated in Fig. 9, the inflatable body portion 240 is formed by laminating a plurality of layers. More specifically, the inflatable body portion 240 includes a first transmission layer 3a and a second transmission layer 3b as the transmission portion 32, and a light absorption layer 3c as the light absorption portion 33.

In the present embodiment, the inflatable member 203 includes the laser emission unit 31. More specifically, in the present embodiment, the laser emission body 241 of the inflatable member 203 includes the laser emission unit 31. The laser emission body 241 may be, for example, a nanosecond pulse laser or a femtosecond pulse laser. The laser emission body 241 is attached to an inner surface of inflatable body portion 240. The plurality of laser emission bodies 241 of the present embodiment is disposed at different positions in the longitudinal direction A and the circumferential direction B of the inflatable body portion 240. The laser emission unit 31 of the laser emission body 241 can emit a laser beam from the inner surface side of the inflatable body portion 240 toward the outside in the radial direction C.

As illustrated in Fig. 9, a plurality of laser emission bodies 241 including the laser emission unit 31 is disposed at intervals in the circumferential direction B of the elongated member 202. In this way, the laser beam can be emitted from the plurality of laser emission units 31 of the plurality of laser emission bodies 241 toward the outside in the radial direction C in a wide range in the circumferential direction B. As a result, the laser-induced shock wave can more reliably act on the calcified area X not only when the calcified area X is formed over the entire circumferential area of the inner wall of the blood vessel BV but also when the calcified area X is formed only in a part of the circumferential area of the inner wall of the blood vessel BV.

In addition, similarly to the laser emission body 20 of the first embodiment (see Fig. 2 and the like), it is preferable that a plurality of laser emission bodies 241 including the laser emission unit 31 is arranged at intervals in the longitudinal direction A of the elongated member 202. In addition, the laser emission body 241 may include a laser emission unit 31 that is elongated in the longitudinal direction A and is capable of emitting a wide laser having uniform intensity in the radial direction C regardless of the position in the longitudinal direction A. In such a case, a plurality of laser emission bodies 241 may not be disposed in the longitudinal direction A.

As illustrated in Fig. 9, the laser emission body 241 includes a conveying member 241a including an optical fiber that transmits light to the laser emission unit 31. The arrangement position of the conveying member 241a is not particularly limited, but the conveying member 241a may be disposed along the inner surface of the inflatable body portion 240 as illustrated in Fig. 9.

The laser beam emitted from the laser emission unit 31 of the laser emission body 241 passes through the first transmission layer 3a and the second transmission layer 3b of the inflatable body portion 240, and is absorbed by the light absorption layer 3c. In the light absorption layer 3c, plasma is generated by the absorbed laser beam. The plasma generated in the light absorption layer 3c is likely to stay in the light absorption layer 3c by the first transmission layer 3a and the second transmission layer 3b covering the inside of the light absorption layer 3c in the radial direction C. As a result, a laser-induced shock wave can be sent from the light absorption layer 3c toward the outside in the radial direction C, that is, the outside of the inflatable member 203. In this manner, in the balloon catheter 201 of the present embodiment, it is possible to crush the calcified area X by applying the laser-induced shock wave to the calcified area X (see Fig. 1 and the like) in the blood vessel BV (see Fig. 1 and the like).

It is preferable that at least one of the first transmission layer 3a and the second transmission layer 3b as the transmission portion 32 and the light absorption layer 3c as the light absorption portion 33 extend over the entire region in the circumferential direction B of the elongated member 202. In this way, the laser-induced shock wave can be sent from the light absorption portion 33 to the outside in the radial direction C regardless of the arrangement position of the laser emission unit 31 in the circumferential direction B.

### [Fourth Embodiment]

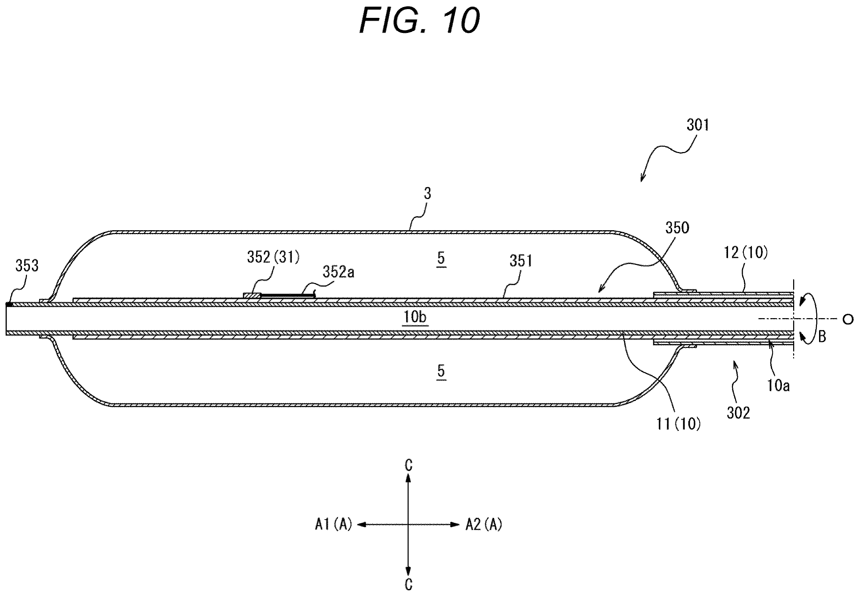

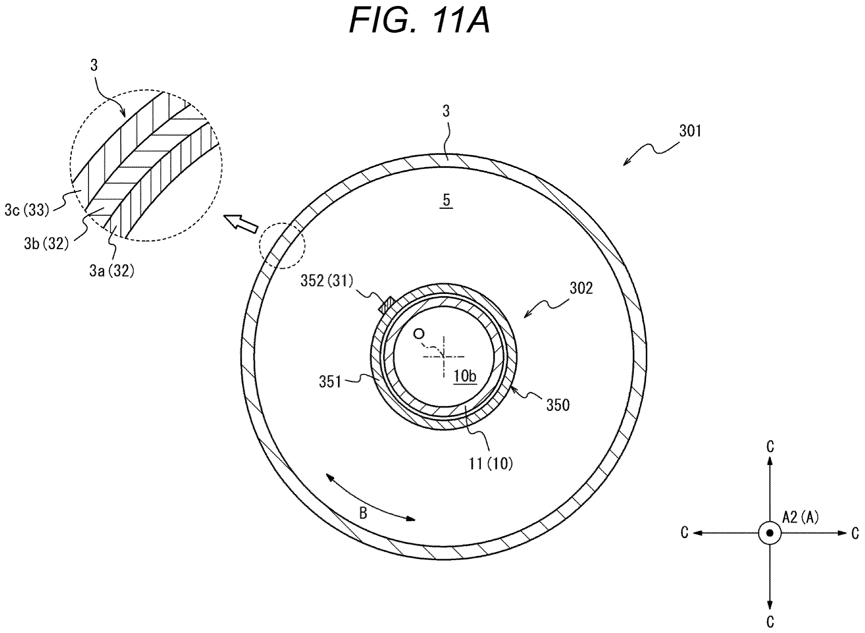

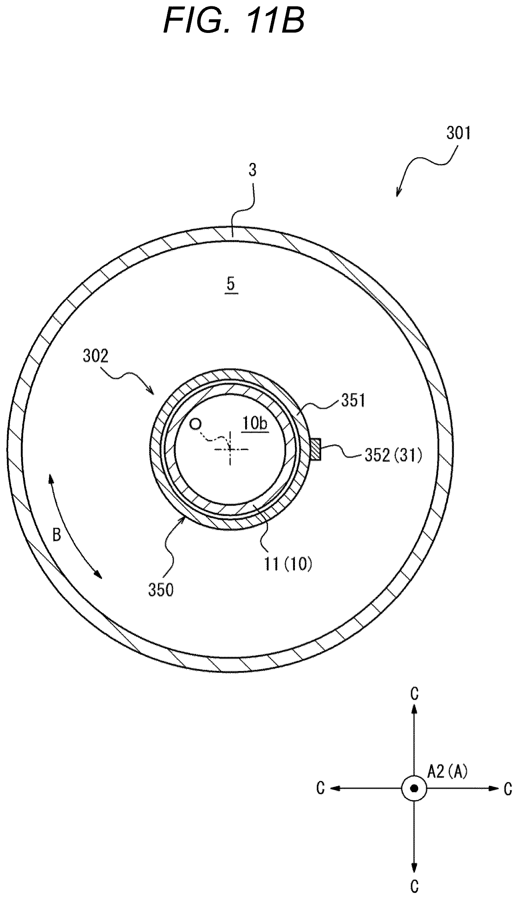

Next, a balloon catheter 301 as another embodiment of the balloon catheter according to the present disclosure will be described with reference to Figs. 10, 11A, and 11B. The balloon catheter 301 of the present embodiment is different from the balloon catheter 1 of the first embodiment described above (see Fig. 1 and the like) in the configuration of the elongated member 302, and the other configurations are common. Here, differences of the balloon catheter 301 from the balloon catheter 1 of the above-described first embodiment (see Fig. 1 and the like) will be described, and description of common configurations will be omitted.

Fig. 10 is an enlarged cross-sectional view illustrating a position of the inflatable member 3 in a cross section of the balloon catheter 301 in a plane including the central axis line O and parallel to the central axis line O in an enlarged manner. Figs. 11A and 11B are cross-sectional views of the balloon catheter 301 in a plane orthogonal to the central axis line O at the position of the inflatable member 3. Fig. 11B illustrates a state in which the sleeve member 350 of the elongated member 302 is relatively rotated in the circumferential direction B with respect to the body member 10 of the elongated member 302 from the state illustrated in Fig. 11A.

As illustrated in Figs. 10, 11A, and 11B, the elongated member 302 of the present embodiment includes a body member 10 and a sleeve member 350. The body member 10 supports the inflatable member 3 on the outer surface. Since the body member 10 has the same configuration as that of the first embodiment described above, the description of the body member 10 will be omitted herein.

The sleeve member 350 is attached to the body member 10 so as to be relatively rotatable in the circumferential direction B with respect to the body member 10. More specifically, the sleeve member 350 surrounds the outer side of the inner tube 11 of the body member 10 in the radial direction C, and is supported on the outer surface of the inner tube 11.

More specifically, the sleeve member 350 of the present embodiment includes an annular sleeve body 351 and a laser emission body 352 attached to the annular sleeve body 351.

The sleeve body 351 is supported on the outer surface of the inner tube 11 so as to be relatively rotatable in the circumferential direction B with respect to the inner tube 11. The sleeve body 351 extends in the longitudinal direction A between the outer surface of the inner tube 11 and the inner surface of the outer tube 12 at a position where the outer tube 12 is provided. The distal end of the sleeve body 351 is located on the proximal side of the outer surface of the inner tube 11 from the position where the inflatable member 3 is joined. More specifically, the distal end of the sleeve body 351 is located in the accommodation space 5 of the inflatable member 3. The sleeve body 351 is slidable with the outer surface of the inner tube 11 in a state of being supported by the outer surface of the inner tube 11, thereby being relatively rotatable in the circumferential direction B with respect to the inner tube 11 and the outer tube 12.

In the present embodiment, the sleeve member 350 includes the laser emission unit 31. More specifically, in the present embodiment, the laser emission body 352 of the sleeve member 350 includes the laser emission unit 31. As illustrated in Figs. 10, 11A, and 11B, the laser emission body 352 of the present embodiment is attached to the sleeve body 351. More specifically, the laser emission body 352 of the present embodiment is attached on the outer surface of the sleeve body 351. Only one laser emission body 352 of the present embodiment is attached on the outer surface of the sleeve body 351. The laser emission body 352 may be, for example, a nanosecond pulse laser or a femtosecond pulse laser. The laser emission unit 31 of the laser emission body 352 can emit a laser beam from the outer surface of the sleeve body 351 toward the outside in the radial direction C.

As described above, the laser emission unit 31 of the present embodiment is provided in the sleeve member 350 that is relatively rotatable with respect to the body member 10. Therefore, the position of the laser emission unit 31 in the circumferential direction B can be changed by relatively rotating the sleeve member 350 with respect to the body member 10. That is, a medical worker such as a doctor can operate the sleeve member 350 to operate the emission direction of the laser beam emitted from the laser emission unit 31. Therefore, for example, when the calcified area X (see Fig. 1 and the like) in the blood vessel BV (see Fig. 1 and the like) is unevenly distributed only in a part of the inner wall of the blood vessel BV in the circumferential direction, the laser emission unit 31 can be aligned with the position of the calcified area X by operating the sleeve member 350. As a result, even in the calcified area X unevenly distributed in a part of the inner wall of the blood vessel BV in the circumferential direction, the laser-induced shock wave can be locally transmitted to the calcified area X.

As illustrated in Fig. 10, the laser emission body 352 includes a conveying member 352a including an optical fiber that transmits light to the laser emission unit 31. The arrangement position of the conveying member 352a is not particularly limited, but the conveying member 352a may be disposed along the outer surface of the sleeve body 351 as illustrated in Fig. 10. In Figs. 11A and 11B, illustration of the conveying member 352a is omitted.

As illustrated in Fig. 10, in the balloon catheter 301 of the present embodiment, a marker member 353 that displays the position of the laser emission unit 31 in the circumferential direction B is preferably attached to the distal end portion of the inner tube 11 of the body member 10 of the elongated member 302. The marker member 353 has X-ray detectability. Specifically, the marker member 353 is made of a material having high radiopacity. Specifically, the marker member 353 can be made of a material having high radiopacity such as platinum, gold, iridium, or tungsten.

In addition, the sleeve member 350 of the present embodiment is configured to be rotatable in the circumferential direction B with respect to the body member 10 and to be movable in the longitudinal direction A with respect to the body member 10. That is, in the present embodiment, by rotating the sleeve member 350 in the circumferential direction B with respect to the body member 10, the position of the laser emission unit 31 in the circumferential direction B can be varied as described above. Furthermore, in the present embodiment, the position of the laser emission unit 31 in the longitudinal direction A can be changed by moving the sleeve member 350 in the longitudinal direction A with respect to the body member 10. That is, according to the elongated member 302 of the present embodiment, the position of the laser emission unit 31 can be changed in the longitudinal direction A and the circumferential direction B. Therefore, for example, even in a configuration in which only one laser emission body 352 is provided by moving the sleeve member 350 in the longitudinal direction A with respect to the body member 10 while rotating the sleeve member 350 in the circumferential direction B with respect to the body member 10, the laser-induced shock wave can be transmitted to the entire circumferential area of the inner wall of the blood vessel BV over a predetermined range in the extending direction of the blood vessel BV (see Fig. 1 and the like). That is, even in the configuration in which only one laser emission body 352 is provided, the laser-induced shock wave can act on a wide range of the calcified area X (see Fig. 1 and the like) in the blood vessel BV.

The operation of the sleeve member 350 described above may be performed by a medical worker such as a doctor by his/her hand, or may be electrically performed using a drive device.

In addition, the sleeve member 350 of the present embodiment includes only one laser emission body 352, but is not limited to this configuration. The sleeve member 350 may comprise a plurality of laser emission bodies 352, each comprising a separate laser emission unit 31. The sleeve member 350 may include, for example, a plurality of laser emission bodies 352 at different positions in the circumferential direction B. For example, the plurality of laser emission bodies 352 may be arranged at equal intervals over the entire region in the circumferential direction B. In such a case, for example, by moving the sleeve member 350 in the longitudinal direction A with respect to the body member 10 without rotating the sleeve member 350 in the circumferential direction B with respect to the body member 10, the laser-induced shock wave can be sent to the entire circumferential area of the inner wall of the blood vessel BV over a predetermined range in the extending direction of the blood vessel BV (see Fig. 1 and the like).

Furthermore, in the inflatable member 3, it is preferable that at least one of the first transmission layer 3a and the second transmission layer 3b as the transmission portion 32 and the light absorption layer 3c as the light absorption portion 33 extend over the entire region in the circumferential direction B of the elongated member 302. In this way, by rotating the sleeve member 350, the laser-induced shock wave can be transmitted from the light absorption portion 33 to the outside in the radial direction C in the entire region in the circumferential direction B.

As illustrated in Fig. 11A, in the present embodiment, the sleeve member 350 includes the laser emission unit 31, and the inflatable member 3 includes the transmission portion 32 and the light absorption portion 33, but the present disclosure is not limited to this configuration. The sleeve member may include the laser emission unit 31, the transmission portion 32, and the light absorption portion 33 (see Fig. 12).

### Fifth Embodiment

Next, a balloon catheter 401 as another embodiment of the balloon catheter according to the present disclosure will be described with reference to Fig. 12. The balloon catheter 401 of the present embodiment is different from the balloon catheter 301 of the above-described fourth embodiment (see Fig. 10 and the like) in the configurations of the elongated member 402 and the inflatable member 403. Here, differences between the balloon catheter 401 and the balloon catheter 301 (see Fig. 10 and the like) of the above-described fourth embodiment will be described, and description of common configurations will be omitted.

Fig. 12 is a cross-sectional view of the balloon catheter 401 in a plane orthogonal to the central axis line O at the position of the inflatable member 403. As illustrated in Fig. 12, the balloon catheter 401 includes an elongated member 402 and an inflatable member 403. The inflatable member 403 is supported on the outer surface of the elongated member 402, and is inflatable outward in the radial direction C by supplying a fluid to the accommodation space 5. Fig. 12 illustrates the inflatable member 403 in the inflated state. Since the inflatable member 403 of the present embodiment has the same configuration as the inflatable member 103 of the second embodiment described above, the description of the inflatable member 403 will be omitted here.

The elongated member 402 includes a body member 10 and a sleeve member 450. Since the body member 10 has the same configuration as that of the fourth embodiment, the description of the body member 10 is omitted here.

The sleeve member 450 of the present embodiment includes a laser emission unit 31, a transmission portion 32, and a light absorption portion 33.

Specifically, the sleeve member 450 of the present embodiment includes a sleeve body 451 and a laser emission body 452. The sleeve body 451 of the present embodiment includes a base material layer 451a, a transmission layer 451b as the transmission portion 32 laminated on the outer side in the radial direction C of the base material layer 451a, and a light absorption layer 451c as the light absorption portion 33 laminated on the outer side in the radial direction C of the transmission layer 451b.

The base material layer 451a may be made of, for example, the materials exemplified as the materials for forming the inner tube 11 (see Fig. 3 and the like) of the first embodiment described above.

The transmission layer 451b may be made of, for example, the materials exemplified as the materials for forming the first transmission layer 3a and the second transmission layer 3b (see Fig. 6) of the inflatable member 3 of the first embodiment described above.

The light absorption layer 451c may be made of, for example, the materials exemplified as the materials for forming the light absorption layer 3c (see Fig. 6) of the inflatable member 3 of the first embodiment described above.

The laser emission body 452 of the present embodiment is embedded in the peripheral wall of the sleeve body 451. More specifically, the laser emission body 452 of the present embodiment is sandwiched between the base material layer 451a and the transmission layer 451b. The laser emission unit 31 of the laser emission body 452 can emit a laser beam from the outer surface of the base material layer 451a of the sleeve body 451 toward the outside in the radial direction C.

As illustrated in Fig. 12, the laser emission body 452 includes a conveying member 452a including an optical fiber that transmits light to the laser emission unit 31. The arrangement position of the conveying member 452a is not particularly limited, but the conveying member 452a may be disposed along the inner surface of the sleeve body 451, for example, as illustrated in Fig. 12.

The laser beam emitted from the laser emission unit 31 of the laser emission body 452 passes through the transmission layer 451b and is absorbed by the light absorption layer 451c. In the light absorption layer 451c, plasma is generated by the absorbed laser beam. The plasma generated in the light absorption layer 451c is likely to stay in the light absorption layer 451c by the transmission layer 451b covering the inside of the light absorption layer 451c in the radial direction C. As a result, the laser-induced shock wave can be transmitted from the light absorption layer 451c toward the outside in the radial direction C, that is, the outside of the sleeve member 450. The laser-induced shock wave propagates through the fluid accommodated in the accommodation space 5 and the inflatable member 403, and is transmitted to the outside of the inflatable member 403 in the radial direction C. In this manner, in the balloon catheter 401 of the present embodiment, it is possible to crush the calcified area X by applying the laser-induced shock wave to the calcified area X (see Fig. 1 and the like) in the blood vessel BV (see Fig. 1 and the like).

In the present embodiment, in the sleeve body 451, the transmission layer 451b as the transmission portion 32 and the light absorption layer 451c as the light absorption portion 33 preferably extend over the entire region in the circumferential direction B of the elongated member 402. In this way, the laser-induced shock wave can be transmitted from the light absorption portion 33 to the outside in the radial direction C regardless of the arrangement position of the laser emission unit 31 in the circumferential direction B with respect to the sleeve body 451.

The sleeve body 451 of the sleeve member 450 of the present embodiment has a three-layer configuration of the base material layer 451a, the transmission layer 451b, and the light absorption layer 451c, but is not limited to this configuration. The sleeve body 451 may have, for example, a two-layer configuration including only the transmission layer 451b and the light absorption layer 451c. In such a case, the laser emission body 452 may be attached to the inner surface of the transmission layer 451b constituting the inner surface layer of the sleeve body 451. Furthermore, the sleeve body 451 may have a configuration of four or more layers including the transmission layer 451b and the light absorption layer 451c, for example. Even in such a case, the sleeve body 451 includes only at least one transmission layer including the transmission layer 451b inside the light absorption layer 451c in the radial direction C, and does not include a layer other than the transmission layer. Further, in the sleeve body 451 of the present embodiment, the light absorption layer 451c as the light absorption portion 33 is the outer surface layer of the sleeve body 451, but another transmission layer may be laminated on the outer side in the radial direction C. However, as in the present embodiment, the light absorption layer 451c is preferably an outer surface layer of the sleeve body 451. In this way, it is possible to suppress attenuation of the laser-induced shock wave by another transmission layer on the outer side in the radial direction C of the light absorption layer 451c.

The balloon catheter according to the present disclosure is not limited to the specific configurations illustrated in the above-described embodiments, and various changes, modifications, and combinations can be made without departing from the scope of the claims.

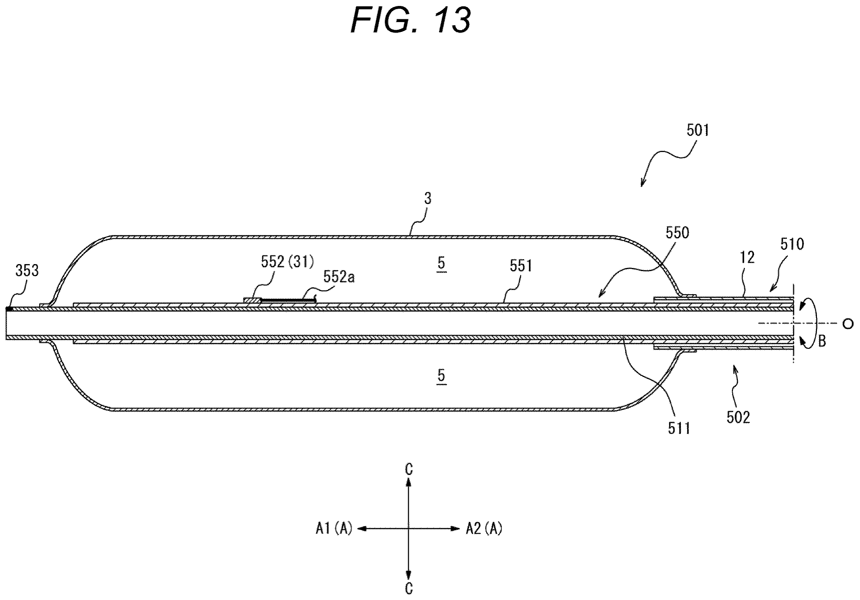

Fig. 13 is a view illustrating a balloon catheter 301 as a modification of the balloon catheter 501 as the above-described fourth embodiment. The balloon catheter 501 illustrated in Fig. 13 is different from the balloon catheter 301 as the above-described fourth embodiment in that the laser emission body 552 has a configuration of a full circumferential irradiation type, and that the sleeve body 551 of the sleeve member 550 of the elongated member 502 and the inner tube 511 of the body member 510 of the elongated member 502 are configured to transmit the laser beam emitted from the laser emission body 552, and other configurations are similar. Therefore, only the above difference will be described here.

The full circumferential irradiation type laser emission body 552 may be, for example, a radial fiber. According to the full circumferential irradiation type laser emission body 552, the laser beam can be emitted radially in the entire region in the circumferential direction B with the laser emission body 552 as the center. The laser emission body 552 includes a conveying member 552a including an optical fiber that transmits light to the laser emission unit 31.

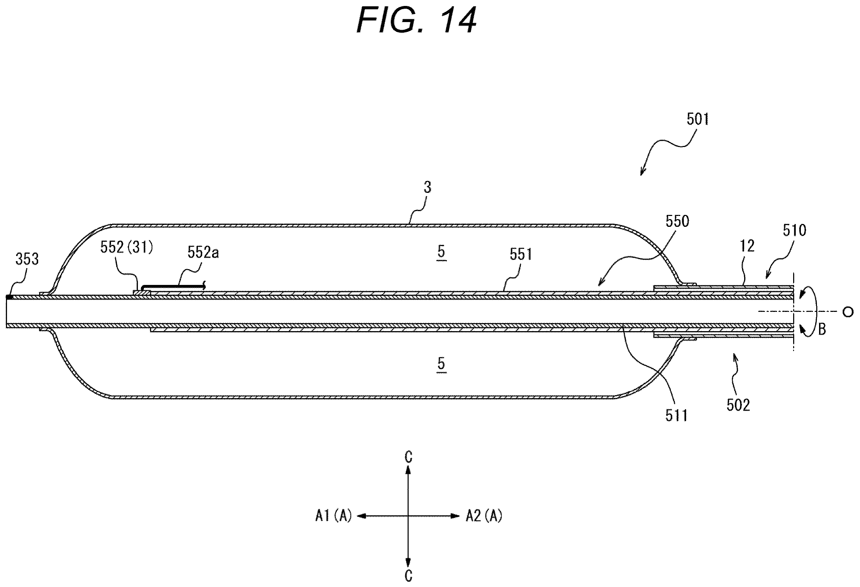

As illustrated in Fig. 13, only one full circumferential irradiation type laser emission body 552 of this example is attached to a part of the sleeve body 551 in the circumferential direction B. Therefore, in the region of the inflatable member 3 where the sleeve body 551 of the sleeve member 550 and the inner tube 511 of the body member 510 are interposed between the inflatable member 3 and the laser emission unit 31 of the laser emission body 552, the laser from the laser emission unit 31 of the laser emission body 552 hardly reaches the region of the inflatable member 3 where the sleeve body 551 of the sleeve member 550 and the inner tube 511 of the body member 510 are interposed between the inflatable member 3 and the laser emission unit 31 of the laser emission body 552. Therefore, in the sleeve member 550 and the body member 510, a portion interposed between the laser emission unit 31 and the inflatable member 3 is configured to transmit the laser beam emitted from the laser emission unit 31. Specifically, in the present example, in the sleeve body 551 of the sleeve member 550 and the inner tube 511 of the body member 510, a portion interposed between the laser emission body 552 and the inflatable member 3 is configured to transmit the laser beam emitted from the laser emission unit 31. In this way, the radial laser beam emitted from the laser emission unit 31 of the laser emission body 552 can easily reach the entire region in the circumferential direction B of the inflatable member 3. In the present example, a part of both the sleeve member 550 and the body member 510 is configured to transmit the laser beam emitted from the laser emission unit 31, but the present disclosure is not limited to this configuration. When only one of the sleeve member 550 and the body member 510 is interposed between the laser emission unit 31 and the inflatable member 3, only one of the sleeve member and the body member may be configured to transmit the laser beam emitted from the laser emission unit 31 (see Fig. 14).

The configurations of the sleeve body 551 and the inner tube 511 are not particularly limited as long as the laser beam emitted from the laser emission body 552 can pass the sleeve body 551 and the inner tube 511. The sleeve body 551 and the inner tube 511 can be exemplified by, for example, a transparent configuration formed of a polymer material such as polyolefins (for example, polyethylene, polypropylene, polybutene, ethylene-propylene copolymer, ethylene-vinyl acetate copolymer, ionomer, or a mixture of two or more types of the polyolefins listed above), polyvinyl chloride, polyamide, polyamide elastomer, polyurethane, polyurethane elastomer, polyimide, or fluororesin, or a mixture of the polymer materials listed above.

With such a configuration, even in a configuration in which only one laser emission body 552 is provided, by moving the sleeve member 550 in the longitudinal direction A with respect to the body member 510 without rotating the sleeve member 550 in the circumferential direction B with respect to the body member 510, the laser-induced shock wave can be transmitted to the entire circumferential area of the inner wall of the blood vessel BV over a predetermined range in the extending direction of the blood vessel BV (see Fig. 1 and the like). Therefore, in the present example, the sleeve member 550 may be configured not to rotate in the circumferential direction B with respect to the body member 510.

However, the sleeve member 550 may be configured to be rotatable in the circumferential direction B with respect to the body member 510. In the present example, only one full circumferential irradiation type laser emission body 552 is provided, but the present disclosure is not limited to this configuration. A plurality of the full circumferential irradiation type laser emission bodies 552 may be provided at different positions in the circumferential direction B, for example, two laser emission bodies may be disposed at opposing positions in the radial direction C.

In addition, Fig. 13 illustrates an example in which the full circumferential irradiation type laser emission body 552 is attached on the outer surface on the outer side in the radial direction C of the sleeve body 551, but the present disclosure is not limited to this configuration. As illustrated in Fig. 14, the full circumferential irradiation type laser emission body 552 may be attached to the distal end portion of the sleeve body 551 so as to protrude in the distal direction A1 from the distal end of the sleeve body 551, for example. In such a case, at least the inner tube 511 of the sleeve body 551 and the inner tube 511 may be configured to transmit the laser beam emitted from the laser emission body 552.

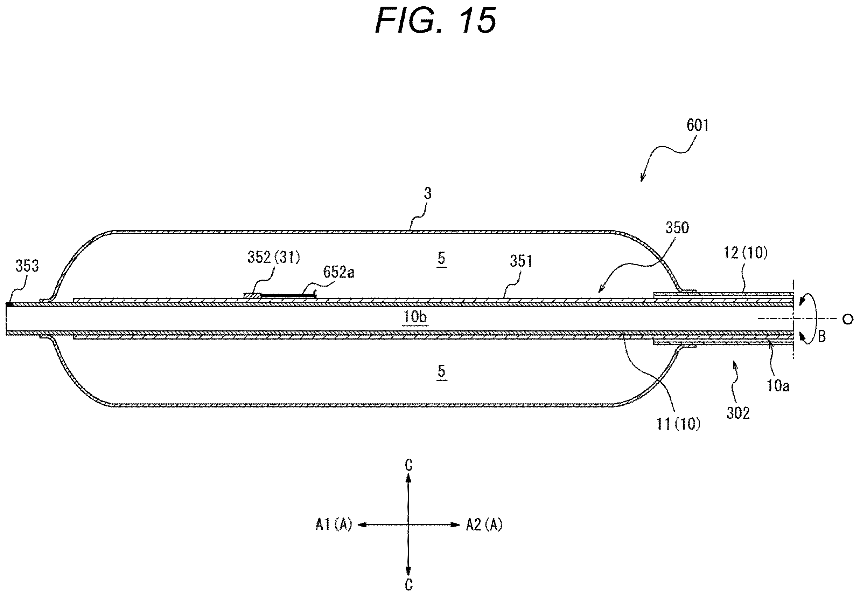

Furthermore, Fig. 15 is a view illustrating a balloon catheter 301 as another modification of the balloon catheter 601 as the above-described fourth embodiment. The balloon catheter 601 illustrated in Fig. 15 is different from the balloon catheter 301 as the fourth embodiment described above in that the conveying member 652a that transmits light to the laser emission unit 31 of the laser emission body 352 has a structure capable of transmitting laser beams of different outputs, more specifically, the conveying member 652a is a double-cladded fiber, and other configurations are similar. Therefore, only the above difference will be described here.

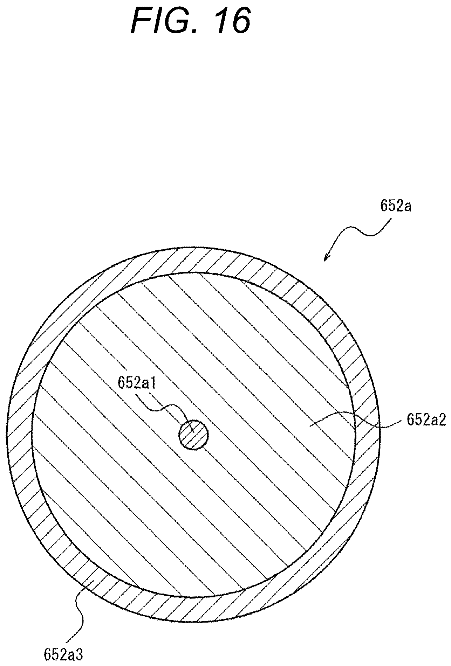

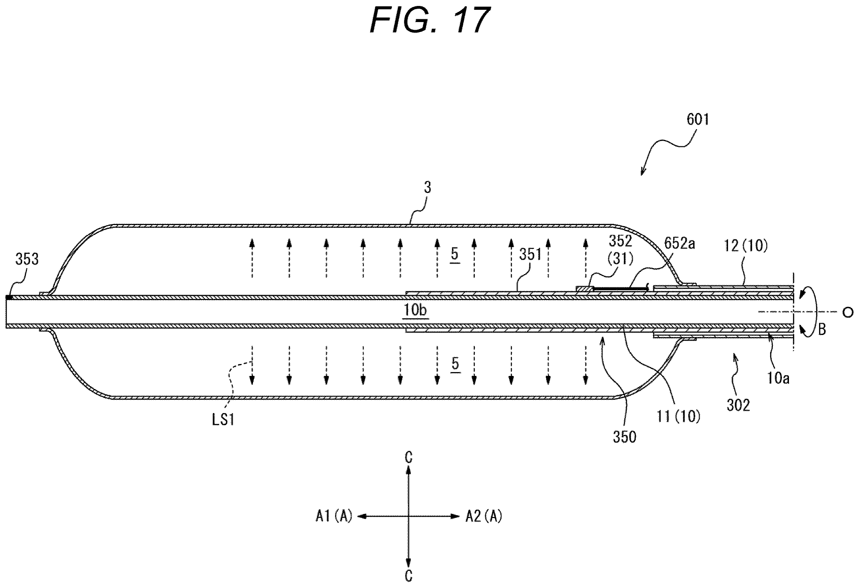

Fig. 16 is a cross-sectional view of a double-cladded fiber as the conveying member 652a. Figs. 17 to 19 are views illustrating an example of a procedure of continuously performing crushing of a calcified area X (see Fig. 1) as treatment of a target site and diagnosis of the target site after the treatment using the balloon catheter 601 illustrated in Fig. 15.

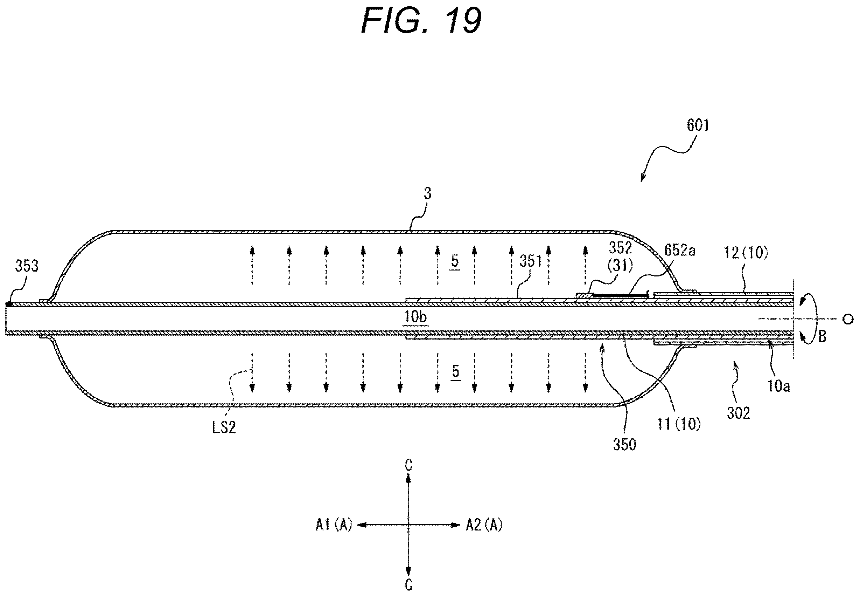

As illustrated in Fig. 16, the double-cladded fiber as the conveying member 652a includes a core 652a1, an inner cladding 652a2 covering the radial outside of the core 652a1, and an outer cladding 652a3 covering the radial outside of the inner cladding 652a2. The refractive index of the core 652a1 is higher than the refractive index of the inner cladding 652a2 and higher than the refractive index of the outer cladding 652a3. The refractive index of the inner cladding 652a2 is higher than the refractive index of the outer cladding 652a3. According to such a double-cladded fiber, a high-power treatment laser beam LS1 (see Fig. 17) can be propagated in the inner cladding 652a2, and a diagnostic laser beam LS2 (see Fig. 19) can be propagated in the core 652a1. In other words, the laser emission unit 31 can be used for emission of the treatment laser beam LS1, and can also be used for transmission and reception of the diagnostic laser beam LS2 used for acquisition of a diagnosis image of a target site after treatment by optical coherence tomography (OCT) or optical frequency domain imaging (OFDI).

Next, an example of a procedure for continuously performing crushing of the calcified area X (see Fig. 1) as treatment of the target site and diagnosis of the target site after the treatment using the balloon catheter 601 will be described with reference to Figs. 17 to 19. Fig. 17 is a diagram illustrating a state in which the treatment of the target site by the treatment laser beam LS1 is completed from the state illustrated in Fig. 15. More specifically, Fig. 17 is a view illustrating a state in which the sleeve member 350 is moved in the proximal direction A2 in the longitudinal direction A with respect to the body member 10 while the sleeve member 350 is rotated in the circumferential direction B with respect to the body member 10 from the state illustrated in Fig. 15. During this movement, a laser-induced shock wave can be transmitted to the entire circumferential area of the inner wall of the blood vessel BV over a predetermined range in the extending direction of the blood vessel BV (see Fig. 1 and the like) by the treatment laser beam LS1 emitted from the laser emission unit 31. In Fig. 17, the treatment laser beam LS1 emitted while the sleeve member 350 is moving from the position illustrated in Fig. 15 to the position illustrated in Fig. 17 is indicated by a broken line. As a result, it is possible to perform crushing of the calcified area X (see Fig. 1) in the blood vessel BV as treatment of the target site.

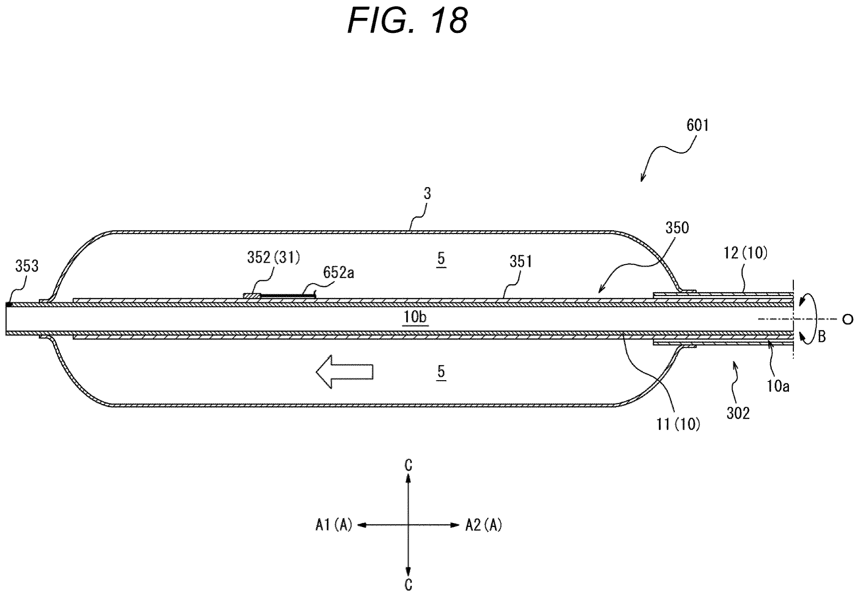

Fig. 18 is a view illustrating a state in which the sleeve member 350 is returned from the position illustrated in Fig. 17 to the position illustrated in Fig. 15. More specifically, Fig. 18 is a view illustrating a state in which the sleeve member 350 is moved in the distal direction A1 of the longitudinal direction A with respect to the body member 10 from the state illustrated in Fig. 17 and returned to the same position as in Fig. 16. During this movement, the treatment laser beam LS1 (see Fig. 17) and the diagnostic laser beam LS2 (see Fig. 19) are not emitted from the laser emission unit 31. During this movement, the sleeve member 350 is moved in the distal direction A1 without pivoting in the circumferential direction B with respect to the body member 10.

Fig. 19 is a diagram illustrating a state in which transmission and reception of the diagnostic laser beam LS2 has been completed in order to acquire the diagnosis image for the target site after the treatment from the state illustrated in Fig. 18. More specifically, Fig. 19 is a view illustrating a state in which the sleeve member 350 is moved again in the proximal direction A2 in the longitudinal direction A with respect to the body member 10 while the sleeve member 350 is rotated in the circumferential direction B with respect to the body member 10 from the state illustrated in Fig. 18. During this movement, the diagnostic laser beam LS2 is transmitted and received by the laser emission unit 31. In Fig. 19, the diagnostic laser beam LS2 transmitted and received while the sleeve member 350 moves from the position illustrated in Fig. 18 to the position illustrated in Fig. 19 is indicated by a broken line. As a result, a diagnosis image of a target site after treatment can be acquired. That is, according to the balloon catheter 601, after the treatment of the target site, the diagnosis image of the target site after the treatment can be acquired without removing the balloon catheter 601 from the living body.

As described above, the treatment of the target site and the diagnosis of the target site after the treatment can be continuously performed by using the double-cladded fiber as the conveying member 652a.

### Industrial Applicability

The present disclosure relates to a balloon catheter.

### Reference Signs List

1 Balloon catheter
2 Elongated member
3 Inflatable member
3a First transmission layer (example of transmission portion)
3b Second transmission layer (example of transmission portion)
3c Light absorption layer (example of light absorption portion)
4 Hub
4a Connection portion
4b Proximal opening
5 Accommodation space
10 Body member
10a Flow path
10b Guide wire insertion hole
11 Inner tube
12 Outer tube
13 Marker member
20 Laser emission body
20a Conveying member
31 Laser emission unit
32 Transmission portion
33 Light absorption portion
101 Balloon catheter
102 Elongated member
103 Inflatable member
110 Body member
110b Guide wire insertion hole
111 Inner tube
111a Base material layer
111b Transmission layer (example of transmission portion)
111c Light absorption layer (example of light absorption portion)
201 Balloon catheter
202 Elongated member
203 Inflatable member
240 Inflatable body portion
241 Laser emission body
241a Conveying member
301 Balloon catheter
302 Elongated member
350 Sleeve member
351 Sleeve body
352 Laser emission body
352a Conveying member
353 Marker member
401 Balloon catheter
402 Elongated member
403 Inflatable member
450 Sleeve member
451 Sleeve body
451a Base material layer
451b Transmission layer (example of transmission portion)
451c Light absorption layer (example of light absorption portion)
452 Laser emission body
452a Conveying member
501 Balloon catheter
502 Elongated member
510 Body member
511 Inner tube
550 Sleeve member
551 Sleeve body
552 Laser emission body
552a Conveying member
601 Balloon catheter
652a Conveying member
652a1 Core
652a2 Inner cladding
652a3 Outer cladding
A Longitudinal direction
A1 Distal direction
A2 Proximal direction
B Circumferential direction
C Radial direction
BV Blood vessel
L1 Irradiation range irradiated with laser beam from laser emission unit in light absorption portion

### Contact region

LS1 Treatment laser beam
LS2 Diagnostic laser beam
0 Central axis line
X Calcified area

## Claims

1. A balloon catheter comprising:
an elongated member;
an inflatable member supported on an outer surface of the elongated member and inflatable outward in a radial direction of the elongated member; wherein
from an inner side to an outer side in the radial direction of the balloon catheter, the balloon catheter further includes:
a laser emission unit that can emit a laser beam outward in the radial direction,
a transmission portion that can transmit the laser beam emitted from the laser emission unit in the radial direction, and
a light absorption portion that can absorb the laser beam having passed through the transmission portion are provided; and
the laser emission unit, the transmission portion, and the light absorption portion are provided only in the elongated member, only in the inflatable member, or in both the elongated member and the inflatable member in a divided manner.

2. The balloon catheter according to claim 1, wherein
the elongated member includes the laser emission unit; and
the inflatable member includes the transmission portion and the light absorption portion.

3. The balloon catheter according to claim 2, wherein
the elongated member includes
a body member supporting the inflatable member on an outer surface, and
a sleeve member attached to the body member so as to be rotatable in a circumferential direction relative to the body member; and
the sleeve member includes the laser emission unit.

4. The balloon catheter according to claim 3, wherein the sleeve member is attached to the body member so as to be movable relative to the body member in a longitudinal direction of the elongated member.

5. The balloon catheter according to claim 2, wherein
the elongated member includes
a body member supporting the inflatable member on an outer surface, and
a sleeve member attached to the body member;
the sleeve member includes the laser emission unit; and
the laser emission unit is of a full circumferential irradiation type configured to emit a radial laser beam.

6. The balloon catheter according to claim 5, wherein a portion of the sleeve member and/or the body member interposed between the laser emission unit and the inflatable member is configured to transmit a laser beam emitted from the laser emission unit.

7. The balloon catheter according to claim 1, further comprising: a conveying member that is continuous with the laser emission unit and conveys light is provided, and wherein the conveying member is configured to convey laser beams of different powers.

8. The balloon catheter according to claim 7, wherein the conveying member is double-cladded fiber, and wherein the double-cladded fiber is configured to propagate a treatment laser beam as well as a diagnostic laser beam.

9. The balloon catheter according to any one of claims 2 to 4, wherein
the inflatable member includes:
a transmission layer as the transmission portion, the transmission layer being capable of transmitting the laser beam in the radial direction; and
a light absorption layer as the light absorption portion that is located outward in the radial direction with respect to the transmission layer and absorbs the laser beam having passed through the transmission layer.

10. The balloon catheter according to claim 1, wherein the elongated member includes the laser emission unit, the transmission portion, and the light absorption portion.

11. The balloon catheter according to claim 10, wherein the elongated member includes:
a body member supporting the inflatable member on an outer surface, and
a sleeve member attached to the body member so as to be rotatable in a circumferential direction relative to the body member; and
the sleeve member includes the laser emission unit, the transmission portion, and the light absorption portion.

12. The balloon catheter according to claim 11, wherein the sleeve member is attached to the body member so as to be movable relative to the body member in a longitudinal direction of the elongated member.

13. The balloon catheter according to claim 1, wherein the inflatable member includes the laser emission unit, the transmission portion, and the light absorption portion.

14. The balloon catheter according to claim 13, wherein
the inflatable member includes
an inflatable body portion formed by laminating a plurality of layers, and
a laser emission body attached to the inflatable body portion;
the laser emission body includes the laser emission unit; and
the inflatable body portion includes
a transmission layer as the transmission portion, the transmission layer being capable of transmitting the laser beam in the radial direction, and
a light absorption layer as the light absorption portion that is located outward in the radial direction with respect to the transmission layer and absorbs the laser beam having passed through the transmission layer.

15. The balloon catheter according to any one of claims 1 to 4, wherein the transmission portion and the light absorption portion extend over the entire area of the elongated member in a circumferential direction.

16. The balloon catheter according to any one of claims 1 to 4, wherein a plurality of the laser emission units is arranged at intervals in a circumferential direction of the elongated member.
